(19) Europäisches Patentamt
European Patent Office
Office européen des brevets

(11) EP 2 147 043 B1

(12) EUROPÄISCHE PATENTSCHRIFT

(45) Veröffentlichungstag und Bekanntmachung des Hinweises auf die Patenterteilung:
**27.02.2019 Patentblatt 2019/09**

(21) Anmeldenummer: **08773345.7**

(22) Anmeldetag: **21.05.2008**

(51) Int Cl.:
***C08J 3/24*** (2006.01) ***B01F 9/02*** (2006.01)
***A61L 15/60*** (2006.01) ***B01F 9/06*** (2006.01)
***B01F 9/00*** (2006.01)

(86) Internationale Anmeldenummer:
**PCT/EP2008/004085**

(87) Internationale Veröffentlichungsnummer:
**WO 2008/141821 (27.11.2008 Gazette 2008/48)**

(54) **VERFAHREN ZUM SCHONENDEN MISCHEN UND BESCHICHTEN VON SUPERABSORBERN**
METHOD FOR GENTLY MIXING AND COATING SUPERABSORBERS
PROCÉDÉ POUR LE MÉLANGE ET LE REVÊTEMENT DÉLICATS DE SUPERABSORBANTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.05.2007 DE 102007024080**

(43) Veröffentlichungstag der Anmeldung:
**27.01.2010 Patentblatt 2010/04**

(73) Patentinhaber: **Evonik Degussa GmbH**
**45128 Essen (DE)**

(72) Erfinder:
- **HARREN, Jörg**
**47807 Krefeld (DE)**
- **Vorholt, Herbert**
**45721 Haltern am See (DE)**
- **VAN STIPHOUDT, Manfred**
**47906 Kempen (DE)**
- **Hose, Rüdiger**
**47918 Tönisvorst (DE)**
- **RAMLOW, Stephan**
**47807 Krefeld (DE)**
- **DERSTAPPEN, Stefan**
**47918 Tönisvorst (DE)**
- **BUSCH, Axel**
**47805 Krefeld (DE)**
- **LANGE, Michael**
**47495 Rheinberg (DE)**
- **INGER, Waldemar**
**47829 Krefeld (DE)**
- **HEBBEN, Kai**
**47918 Tönisvorst (DE)**

(74) Vertreter: **Lang, Arne et al**
**Evonik Industries AG**
**Intellectual Property Management**
**Bau 1042A / PB 15**
**Paul-Baumann-Straße 1**
**45764 Marl (DE)**

(56) Entgegenhaltungen:
**EP-A1- 1 462 473**
**EP-A1- 1 757 645**
**EP-A1- 1 757 646**
**WO-A1-00/22017**
**WO-A1-2007/023983**
**WO-A2-2007/024927**
**DE-A1- 19 529 348**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft allgemein ein Verfahren zur Herstellung eines Superabsorbers, eine Vorrichtung zur Herstellung eines Superabsorbers, Superabsorber, einen Verbund, ein Verfahren zur Herstellung eines Verbundes, die durch dieses Verfahren erhältlichen Verbunde, chemische Produkte wie Schäume, Formkörper oder Fasern sowie die Verwendung eines Superabsorbers.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Die Herstellung der Superabsorber erfolgt in der Regel durch die radikalische Polymerisation säuregruppentragender Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

Um die Eigenschaften, insbesondere die Absorptionseigenschaften, der Superabsorber für einen Einsatz in Hygieneartikeln zu optimieren, werden die Polymerpartikel, die nach der Zerkleinerung des getrockneten Polymergels erhalten werden, modifiziert, vorzugsweise Oberflächenmodifiziert. Bei der Oberflächenmodifizierung kann eine Kern-Schale-artige Morphologie entstehen, die insbesondere bei Superabsorberteilchen bevorzugt ist. Diese Modifizierung dient beispielsweise dazu, die Superabsorber mit geruchbindenden Eigenschaften zu versehen, das Staubverhalten der Superabsorber zu verbessern, das Verbacken der Superabsorberpartikel zu vermindern, das Absorptionsvermögen der Superabsorber unter einer Druckbelastung zu verbessern und/oder die Permeabilitätseigenschaften des Superabsorbers vorteilhaft zu beeinflussen.

Bei diesen Modifizierungsmaßnahmen werden die superabsorbierenden Polymerpartikel mit einem Behandlungsmittel, welches in einer Lösung, einer Dispersion oder aber als Pulver vorliegen kann, mit dem Superabsorber in Kontakt gebracht. Um eine möglichst gleichmäßige Behandlung der Oberfläche der Superabsorber mit dem Behandlungsmittel zu erreichen, erfolgt dieses in Kontakt bringen üblicherweise dergestalt, das die Superabsorberpartikel in einem Mischer vorgelegt werden und anschließend das Behandlungsmittel unter intensivem Mischen der Polymerpartikel zugesetzt wird. Im Falle der Oberflächennachvernetzung als O-berflächenmodifizierungsmaßnahme werden hierzu herkömmliche Mischvorrichtungen, wie etwa der Patterson-Kelley-Mischer, der Schugi-Vertikalmischer (z.B. der Firma Hosokawa mit der Handelsbezeichnung "Flexomix"), der DRAIS-Turbulenzmischer, der Lödigemischer, der Ruberg-Mischer, der Schneckenmischer, der Tellermischer oder der Wirbelschichtmischer eingesetzt. Weitere geeignete Mischvorrichtungen sind beispielsweise im Kapitel 3.2.8.1 des Buches "Modern Superabsorbent Polymer Technology" von F.L. Buchholz und A.T. Graham, Wiley-VCH, 1998 beschrieben.

In herkömmlich eingesetzten Mischvorrichtungen neigt bei einem Einsatz wässriger Lösungen als Behandlungsmittel der damit behandelte Superabsorber unmittelbar nach dem Verlassen der Mischvorrichung zum Verklumpen. Auch wird häufig eine inhomogene Verteilung des Behandlungsmittels in bzw. auf dem Superabsorber beobachtet. Um eine solches Verklumpen zu verhindern und die Homogenisierung zu verbessern, wird der Superabsorber nach dem Verlassen der Mischvorrichtung beispielsweise in konischen Silos gelagert und mittels einer umlaufenden Schnecke bewegt (Konusmischer). In einem solchen Konusmischer werden die Superabsorber so lange gelagert, bis sie ausreichend relaxiert und homogenisiert sind und insbesondere von einem erdfeuchten, zum aneinander haften neigenden Zustand in einem freifließenden Zustand überführt worden sind. Eine ausreichende Relaxierung bzw. Homogenisierung ist beispielsweise dann erreicht, wenn der Superabsorber wieder einen frei fließenden Zustand erreicht hat. Dies ist oftmals der Fall, wenn in etwa der gleiche Fließfähigkeits-Wert erreicht wird, den die Superabsorber vor der Zugabe des wässrigen Behandlungsmittels aufgewiesen haben. Zu diesem Zeitpunkt ist die beaufschlagte Flüssigkeit im Wesentlichen homogen innerhalb der Superabsorberpartikel verteilt, so dass der Unterschied zwischen dem Wassergehalt im Kern und dem Wassergehalt im Oberflächenbereich (bei Beaufschlagung mit einem wässrigen Behandlungsmittel) der Polymerpartikel sich zunehmend angleicht.

Nachteilig an derartigen Verfahren ist jedoch, dass eine ausreichende Relaxierung und Homogenisierung nur sehr langsam erzielt werden kann und insbesondere größere Mengen an behandeltem Superabsorber nicht verarbeitet werden können. Durch diese Beschränkungen ist auch die maximale Menge an Flüssigkeit, welche bei der Modifizierung dem Superabsorber zugesetzt werden kann, begrenzt. Darüber hinaus kann es durch die umlaufende Schnecke in den Konusmischern, bzw. in anderen Mischern des Rotor-Stator-Prinzips, leicht zu einer signifikanten mechanischen Schädigung des Superabsorbers kommen, wodurch die Absorptionseigenschaften nachteilig beeinflusst und insbesondere der Staubanteil erhöht wird.

Allgemein liegt der vorliegenden Erfindung die Aufgabe zu Grunde, die sich aus dem Stand der Technik ergebenden Nachteile zu überwinden.

Es ist insbesondere eine Aufgabe der vorliegenden Erfindung, ein Verfahren anzugeben, nach dem absorbierende Polymere hergestellt werden können, wobei eine besonders schnelle und schonende Relaxierung und Homogenisierung eines Superabsorbers, der mit Modifizierungsmitteln, insbesondere mit wässrigen Lösungen als Modifizierungsmittel in Kontakt gebracht worden ist, erreicht werden kann.

Nach einer erfindungsgemäßen Aufgabe sollte dieses Verfahren auch die Herstellung von Superabsorbern unter Einsatz großer Mengen an wässrigen Modifizierungslösungen ermöglichen.

Nach einer weiteren erfindungsgemäßen Aufgabe sollte der Zeitaufwand zum Erreichen einer möglichst homogenen Verteilung an Modifizierungsmitteln bzw. -Lösungen nicht nur zwischen den wasserabsorbierenden Polymergebilden, sondern auch innerhalb dieser verringert werden.

Der vorliegenden Erfindung lag auch die Aufgabe zugrunde, ein Verfahren anzugeben, mit dem es möglich ist, Superabsorber mit möglichst geringem Staubanteil herzustellen.

Der vorliegenden Erfindung lag auch die Aufgabe zugrunde, eine Vorrichtung bereitzustellen, mittels derer Superabsorber auch unter Einsatz großer Flüssigkeitsmengen bei der Modifizierung hergestellt werden können. Auch soll die Vorrichtung die Herstellung möglichst großer Mengen an Superabsorber in einem gegebenen Zeitintervall ermöglichen. Der Staubanteil der durch diese Vorrichtung hergestellten Superabsorber sollte zudem möglichst gering sein.

Allgemein leisten die kategoriebildenden Ansprüche einen Beitrag zur Lösung mindestens einer der vorstehend genannten Aufgaben, wobei die davon abhängigen Ansprüche bevorzugte Ausgestaltungen darstellen.

Einen Beitrag zur Lösung mindestens einer der eingangs genannten Aufgaben leistet ein Verfahren zur Herstellung eines Superabsorbers, beinhaltend als Schritte:

a) Bereitstellen eines wasserabsorbierenden Polymergebildes;
b) in Kontakt bringen des wasserabsorbierenden Polymergebildes mit einem, vorzugsweise wässrigen, Modifizierungsmittel, vorzugsweise einem Oberflächenmodifizierungsmittel;
c) Weiterbehandeln des mit dem Modifizierungsmittel in Kontakt gebrachten, wasserabsorbierenden Polymergebildes;

wobei zumindest die Weiterbehandlung im Verfahrensschritt c) zumindest teilweise in einem sich drehenden Behälter erfolgt.

Im Schritt a) des erfindungsgemäßen Verfahrens wird zunächst ein wasserabsorbierendes Polymergebilde bereitgestellt.

Als im Verfahrensschritt a) bereitgestellte wasserabsorbierende Polymergebilde sind Fasern, Schäume oder Teilchen bevorzugt, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 μm, vorzugsweise 20 bis 2000 μm und besonders bevorzugt 150 bis 850 μm oder 150 bis 600 μm aufweisen. Dabei ist es insbesondere bevorzugt, dass der Anteil der Polymerteilchen mit einer Partikelgröße in einem Bereich von 300 bis 600 μm mindestens 30 Gew.-%, besonders bevorzugt mindestens 40 Gew.-% und am meisten bevorzugt mindestens 50 Gew.-%, bezogen auf das Gesamtgewicht der nachvernetzten, wasserabsorbierenden Polymerteilchen, beträgt.

In einer bevorzugten Ausführungsform der im Verfahrensschritt a) bereitgestellten, wasserabsorbierenden Polymergebilde basieren diese auf

($\alpha$1) 20-99,999 Gew.-%, bevorzugt 55-98,99 Gew.-% und besonders bevorzugt 70-98,79 Gew.-% polymerisierten, ethylenisch ungesättigten, säuregruppentragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,
($\alpha$3) 0,001-5 Gew.-%, vorzugsweise 0,01-3 Gew.-% und besonders bevorzugt 0,01-2,5 Gew.-% eines oder mehrerer Vernetzer,
($\alpha$4) 0-30 Gew.-%, vorzugsweise 0-5 Gew.-% und besonders bevorzugt 0,1-5 Gew.-% eines wasserlöslichen Polymeren,

($\alpha$5) 0-20 Gew.-%, vorzugsweise 2,5-15 Gew.-% und besonders bevorzugt 5-10 Gew.-% Wasser, sowie

($\alpha$6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen ($\alpha$1) bis ($\alpha$6) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere ($\alpha$1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere ($\alpha$1) genannt werden. Besonders bevorzugte ethylenisch ungesättigte, säuregruppenhaltige Monomere ($\alpha$1) sind Acrylsäure und Methacrylsäure, wobei Acrylsäure am meisten bevorzugt ist.

Gemäß einer Ausführungsform des erfindungsgemäßen Verfahrens werden unbehandelte, wasserabsorbierende Polymergebilde eingesetzt, bei denen die monoethylenisch ungesättigten, mit ($\alpha$1) copolymerisierbare Monomere ($\alpha$2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)-acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere ($\alpha$2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit ($\alpha$1) copolymerisierbaren Monomere ($\alpha$2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit ($\alpha$1) copolymerisierbaren Monomere ($\alpha$2) umfassen weiterhin Methylpolyethylenglykolallylether, Vinylacetat, Styrol und Isobutylen.

Als Vernetzer ($\alpha$3) werden vorzugsweise diejenigen Verbindungen eingesetzt, die in der WO 2004/037903 A2 als Vernetzer ($\alpha$3) genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)-acrylate, Triallylmethylammoniumchlorid, Tetraallylammoniumchlorid sowie mit 9 Mol Ethylenoxid pro Mol Acrylsäure hergestelltes Allylnonaethylenglykolacrylat.

Als wasserlösliche Polymere ($\alpha$4) können in den Polymergebilden wasserlösliche Polymere, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich

sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (α6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der im Verfahrensschritt a) bereitgestellten, wasserabsorbierenden Polymergebilde basieren diese zu mindestens 50 Gew.-%, vorzugsweise zu mindestens 70 Gew.-% und darüber hinaus bevorzugt zu mindestens 90 Gew.-% auf carboxylatgruppen-tragenden Monomeren. Es ist erfindungsgemäß weiterhin bevorzugt, dass die Komponente (α1) zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Aus den vorgenannten Monomeren, Comonomeren, Vernetzern, wasserlöslichen Polymeren und Hilfsstoffen lassen sich die unbehandelten, wasserabsorbierenden Polymergebilde durch verschiedene Polymerisationsweisen herstellen. Beispielsweise ist in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren (α1), und (α2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601, DE 28 40 010 und WO 96/05234 A1 beschrieben, deren entsprechende Offenbarung hiermit als Referenz eingeführt wird.

Die bei der Lösungspolymerisation oder der inversen Suspensions- und Emulsionspolymerisation nach der Polymerisation erhaltenen Hydrogele werden in einem weiteren Verfahrenschritt getrocknet.

Insbesondere im Falle der Lösungspolymerisation ist es jedoch bevorzugt, dass die Hydrogele vor der Trocknung zunächst zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels bis zu einem Wassergehalt von 0,5 bis 20 Gew.-%, vorzugsweise von 1 bis 10 Gew.-% erfolgt, wobei die Trocknungstemperaturen üblicherweise in einem Bereich von 100 bis 200°C liegen.

Die nach dem Trocknen erhaltenen wasserabsorbierenden Polymergebilde können, insbesondere dann, wenn

sie durch Lösungspolymerisation erhalten wurden, in einem weiteren Verfahrensschritt noch zermahlen und auf eine die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa Walzenstuhl oder Schneidmühle.

Gemäß einer anderen, bevorzugten Ausführungsform sind die im Verfahrensschritt a) eingesetzten, wasserabsorbierenden Polymergebilde bereits oberflächennachvernetzt. Bei der Oberflächennachvernetzung werden die getrockneten Polymergebilde oder aber das noch nicht getrocknete, jedoch vorzugsweise bereits zerkleinerte Hydrogel mit einem vorzugsweise organischen, chemischen Oberflächennachvernetzer in Kontakt gebracht. Dabei wird der Nachvernetzer insbesondere dann, wenn er unter den Nachvernetzungsbedingungen nicht flüssig ist, vorzugsweise in Form eines Fluids $F_1$ umfassend den Nachvernetzer sowie ein Lösungsmittel mit den Polymerteilchen bzw. dem Hydrogel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid $F_1$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids $F_1$, enthalten ist.

Das in Kontakt bringen des Polymergebildes bzw. des zerkleinerten Hydrogels mit dem Fluid $F_1$ beinhaltend den Nachvernetzer erfolgt im erfindungsgemäßen Verfahren vorzugsweise durch gutes Vermischen des Fluids $F_1$ mit dem Polymergebilde.

Geeignete Mischaggregate zum Aufbringen des Fluids $F_1$ sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Fluid $F_1$ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Hydrogele mit dem Nachvernetzer bzw. mit dem Fluid $F_1$ beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Es ist erfindungsgemäß bevorzugt, dass die im Verfahrensschritt a) des erfindungsgemäßen Verfahrens bereitgestellten, wasserabsorbierenden Polymergebilde mindestens eine der folgenden Eigenschaften, vorzugsweise alle folgenden Eigenschaften aufweisen:

(β1) die maximale Aufnahme von 0,9 gew.-%iger, wässriger NaCl-Lösung gemäß ERT 440.2-02 liegt in einem Bereich von mindestens 10 bis 1000, bevorzugt von 15 bis 500 und besonders bevorzugt von 20 bis 70 g/g;

(β2) der mit 0,9 gew.-%iger, wässriger NaCl-Lösung extrahierbare Anteil gemäß ERT 470.2-02 beträgt weniger als 30, bevorzugt weniger als 20 und besonders bevorzugt weniger als 10 Gew.-%, bezogen auf das absorbierende Polymer;

(β3) die Schüttdichte gemäß ERT 460.2-02 liegt im Bereich von 300 bis 1000, bevorzugt 310 bis 800 und besonders bevorzugt 320 bis 700 g/l,

(β4) der pH-Wert gemäß ERT 400.2-02 von 1 g des absorbierenden Polymers in 1 l Wasser liegt im Bereich von 4 bis 10, bevorzugt von 5 bis 9 und besonders bevorzugt von 5,5 bis 7,5,

(β5) der CRC-Wert nach ERT 441.2-02 liegt im Bereich von 10 bis 100, bevorzugt 15 bis 80 und besonders bevorzugt 20 bis 60 g/g,

(β6) der AAP-Wert gemäß ERT 442.2-02 bei einem Druck von 20 g/cm$^2$ liegt im Bereich von 10 bis 60, bevorzugt 15 bis 50 und besonders bevorzugt 20 bis 40 g/g.

Die sich aus den vorstehenden Eigenschaften ergebenden Eigenschaftskombinationen von zwei oder mehr dieser Eigenschaften stellen jeweils bevorzugte Ausführungsformen des im Verfahrensschritt a) bereitgestellten, wasserabsorbierenden Polymergebildes dar. Weiterhin als erfindungsgemäße Ausführungsformen besonders bevorzugt sind Verfahren, in denen das im Verfahrensschritt a) eingesetzte, wasserabsorbierende Polymergebilde die nachfolgend als Buchstaben oder Buchstabenkombinationen dargestellten Eigenschaften oder Eigenschaftskombinationen zeigt: (β1), (β2), (β3), (β4), (β5), (β6) und (β1)(β2)(β3)(β4)(β5)(β6).

Im Schritt b) des erfindungsgemäßen Verfahrens werden nun die im Verfahrensschritt a) bereitgestellten, gegebenenfalls bereits oberflächennachvemetzten, wasserabsorbierenden Polymergebilde mit einem Modifizierungsmittel, vorzugsweise mit einem wässrigen Modifizierungsmittel, in Kontakt gebracht.

Gemäß einer bevorzugten Ausführungsform des erfindungsgemäßen Verfahrens handelt es sich bei dem im Verfahrensschritt b) eingesetzten Modifizierungsmittel um ein Mittel ausgewählt ist aus der Gruppe bestehend aus reinen Lösungsmitteln, wie beispielsweise Wasser, einer Lösung, einer Dispersion, einer Emulsion und einem Pulver, wobei der Einsatz reinen Wassers, einer Lösung, einer Dispersion und einer Emulsion besonders bevorzugt und der Einsatz einer Lösung am meisten bevorzugt ist.

Wenn es sich bei dem Modifizierungsmittel um eine Lösung, eine Emulsion oder eine Dispersion handelt, so ist es weiterhin erfindungsgemäß bevorzugt, dass das Modifizierungsmittel zu mindestens 10 Gew.-%, besonders bevorzugt zu mindestens 25 Gew.-% und am meisten bevorzugt zu mindestens 50 Gew.-%, jeweils bezogen auf das Gesamtgewicht des Modifizierungsmittels, aus Wasser besteht.

Eine als Modifizierungsmittel eingesetzte Lösung umfasst mindestens ein gelöstes, modifizierend wirkenden Agens und ein Lösungsmittel. Eine als Modifizierungsmittel eingesetzte Dispersion umfasst mindestens ein dispergiertes, modifizierend wirkendes Agens als Feststoff, ein Dispersionsmittel sowie gegebenenfalls ein Dispergiermittel. Eine als Modifizierungsmittel eingesetzte Emulsion umfasst mindestens ein emulgiertes, modifizierend wirkendes Agens in Form von Flüssigkeitströpfchen, eine flüssige Phase, in der die Flüssigkeitströpfchen verteilt sind sowie gegebenenfalls einen Emulgator.

Als Lösungsmittel, als Dispersionsmittel und als flüssige Phase sind Wasser, mit Wasser mischbare organische Lösungsmittel, wie etwa Methanol, Ethanol, 1,2-Propandiol, 1-Propanol oder 2-Propanol, oder Mischungen aus Wasser und mit Wasser mischbaren, organischen Lösungsmitteln, wie etwa Mischungen aus Wasser und Methanol, Mischungen aus Wasser und Ethanol, Mischungen aus Wasser und 1-Propanol oder Mischungen aus Wasser und 2-Propanol bevorzugt, wobei Wasser am meisten bevorzugt ist.

Als modifizierend wirkendes Agens, die insbesondere als Oberflächenmodifizierungsmittel eingesetzt werden können, können alle dem Fachmann bekannten Verbindungen eingesetzt werden, welche geeignet sind, um insbesondere die Permeabilitäts-, Absorptions-, Retentions-, Staub- oder Fließeigenschaften wasserabsorbierender Polymergebilde zu beeinflussen. Auch Verbindungen, welche antimikrobielle Eigenschaften aufweisen und somit beispielsweise geeignet sind, nach Absorption von Körperflüssigkeiten die Bildung unangenehmer Gerüche zu unterbinden, können als modifizierend wirkendes Agens eingesetzt werden.

- Wenn im Verfahrensschritt a) des erfindungsgemäßen Verfahren nichtoberflächennachvernetzte, wasserabsorbierende Polymergebilde eingesetzt werden, so kann es sich bei dem modifizierend wirkendenden Agens um einen Oberflächennachvernetzter handeln, wobei als Oberflächennachvernetzter diejenigen Verbindungen bevorzugt sind, die bereits eingangs im Zusammenhang mit der Oberflächennachvernetzung als bevorzugt Nachvernetzer genannt wurden.

- Weiterhin kann es sich bei dem modifizierend wirkenden Agens um ein organisches Salz umfassend ein zwei- oder höherwertiges Kation eines Metalls sowie mindestens eine organische Base als Anion handeln. Dabei ist es bevorzugt, dass das zwei- oder höherwertige Kation eines Metalls ausgewählt ist aus der Gruppe bestehend aus $Mg^{2+}$, $Ca^{2+}$, $Ba^{2+}$, $Al^{3+}$, $Fe^{2+}$, $Fe^{3+}$, $Ga^{3+}$, $Ti^{4+}$, $Zr^{4+}$, $Cu^{2+}$ und $Zn^{2+}$, wobei $Al^{3+}$ am meisten bevorzugt ist. Bei der organischen Base handelt es sich vorzugsweise um eine mindestens teilweise deprotonierte Mono-, Di- oder Tricarbonsäure, wobei deprotonierte Monocarbonsäuren besonders bevorzugt sind. Bevorzugt sind weiterhin Hydroxycarbonsäuren, wobei mindestens teilweise deprotonierte Mono-, Di- oder Hydroxytricarbonsäuren bevorzugt und Monohydroxycarbonsäuren besonders bevorzugt sind.
  Besonders bevorzugte Anionen sind insbesondere die korrespondierenden Basen folgender Säuren: Anissäure,

Benzoesäure, Ameisensäure, Valeriansäure, Zitronensäure, Glyoxylsäure, Glykolsäure, Glycerinphosphorsäure, Glutarsäure, Chloressigsäure, Chlorpropionsäure, Zimtsäure, Bernsteinsäure, Essigsäure, Weinsäure, Milchsäure, Brenztraubensäure, Fumarsäure, Propionsäure, 3-Hydroxypropionsäure, Malonsäure, Maleinsäure, Buttersäure, Isobuttersäure, Imidinoessigsäure, Apfelsäure, Isethionsäure, Methylmaleinsäure, Adipinsäure, Itaconsäure, Crotonsäure, Oxalsäure, Salizylsäure, Gallussäure, Sorbinsäure, Gluconsäure, Fettsäuren, insbesondere Stearinsäure und Adipinsäure, und p-Oxybenzoesäure. Unter diesen Basen am meisten bevorzugt sind Tartrat und Lactat, wobei Lactat am allermeisten bevorzugt ist. Das in diesem Zusammenhang am meisten bevorzugt organische Salz ist Aluminiumlactat.

- Auch kann es sich bei dem modifizierend wirkenden Agens um ein anorganisches Salz umfassend ein zwei- oder höherwertiges Kation eines Metalls sowie mindestens ein anorganisches Anion handeln. Als anorganische Salze können alle dem Fachmann bekannten Metallsalze eingesetzt werden, wobei jedoch wasserlösliche Metallsalze besonders bevorzugt sind. Unter "wasserlöslich" werden dabei vorzugsweise Metallsalze verstanden, von denen bei einer Temperatur von 25°C vorzugsweise mindestens 1 g, besonders bevorzugt mindestens 10 g, darüber hinaus bevorzugt mindestens 100 g und am meisten bevorzugt mindestens 500 g in einem Liter destilliertem Wasser löslich sind.
  Unter den vorstehend genannten, vorzugsweise wasserlöslichen Metallsalzen sind insbesondere Sulfate, Sulfite, Sulfide, Chloride, Bromide, Iodide, Nitrate, Nitrite, Phosphate, Phosphite, Carbonate, Hydrogencarbonate, und Hydroxide bevorzugt, wobei Sulfate am meisten bevorzugt sind.
  Weiterhin ist es bevorzugt, dass auch das Metallkation des anorganischen Salzes ein einwertiges, zweiwertiges oder ein dreiwertiges Metallkation ist, wobei auch hier dreiwertige Metallkationen am meisten bevorzugt sind. Als Metallkationen bevorzugt sind $Na^+$, $K^+$, $Li^+$, $Mg^{2+}$, $Ca^{2+}$, $Fe^{2+}$, $Fe^{3+}$, $Ag^+$, $Cu^+$, $Cu^{2+}$, $Zn^{2+}$, sowie $Al^{3+}$, wobei als $Al^{3+}$ am meisten bevorzugt ist.
  Folgende anorganischen Salze sind als modifizierend wirkendes Agens besonders bevorzugt: $AlCl_3 \times 6H_2O$, $NaAl(SO_4)_2 \times 12H_2O$, $Al(NO_3)_3 \times 9H_2O$, $KAl(SO_4)_2 \times 12H_2O$ oder $Al_2(SO_4)_3 \times$ 14-18$H_2O$ sowie die entsprechenden wasserfreien Salze, $Na_2SO_4$ oder dessen Hydrate, $MgSO_4 \times 10H_2O$ oder wasserfreies Magnesiumsulfat.
  In diesem Zusammenhang kann es auch vorteilhaft sein, wenn bei einem Einsatz eines Metallsalzes, insbesondere eines anorganischen Metallsalzes als modifizierend wirkendes Agens dieses in Kombination mit einem Oxid eines Metalls als weiteres modifizierendes Agens eingesetzt wird. Als Oxid eines Metalls können dabei alle Metalloxide eingesetzt werden, wobei als "Oxid eines Metalls" nicht die Oxide von Halbmetallen, wie etwa Bor, Silizium oder Germanium verstanden werden. Besonders bevorzugt beinhalten diese Oxide eines Metalls feinteiliges Zinkoxid, welche zu mindestens 50 Gew.-% auf gegebenenfalls agglomerierten Partikeln mit einer Partikelgröße in einem Bereich von etwa 10 nm bis 500 $\mu$m basiert. Derartige feinteilige Zinkoxide sind beispielsweise unter der Handelbezeichnung "Nanox®" von Elementis Specialities, USA, erhältlich.

- Weiterhin kann es sich bei dem modifizierend wirkenden Agens um ein thermoplastisches Polymer handeln, wobei es in diesem Zusammenhang bevorzugt ist, dass das thermoplastische Polymer in Form einer Polymerdispersion eingesetzt wird. Bevorzugte thermoplastische Polymere sind insbesondere Polymere ausgewählt aus der Gruppe bestehend aus Poly-(meth)acrylaten, (Meth)Acrylsäure-Copolymeren, beispielsweise Ethylen-(Meth)Acrylsäure-Copolymer, (Meth)Acrylsäureester-Copolymeren, Maleinsäure-Copolymeren, beispielsweise Maleinsäure-Propylen-Copolymeren, Polyurethanen, Vinylacetat-Copolymeren, beispielsweise ein EthylenVinylacetat-Copolymeren oder Vinylacetat-Butylacrylat-Copolymeren, Styrol-Copolymeren, beispielsweise Butylacrylat-Styrol-Copolymeren und Polycarbonaten. Dabei steht der Begriff (Meth)acrylsäure für die beiden Verbindungen Methacrylsäure und Acrylsäure, wobei von diesen beiden die Acrylsäure besonders bevorzugt ist. Erfindungsgemäß bevorzugte thermoplastische Polymere sind, was die chemische Zusammensetzung der Polymere angeht, weiterhin all diejenigen thermoplastischen Polymere, die in DE-A-103 34 286 und in WO-A-2005/044900 als thermoplastische Polymere genannt werden. Der Offenbarungsgehalt der DE-A-103 34 286 und der WO-A- 2005/044900 hinsichtlich der dort beschriebenen, thermoplastischen Polymere wird hiermit als Referenz eingeführt und bildet einen Teil der Offenbarung der vorliegenden Erfindung.

- Weiterhin kann es sich bei dem modifizierend wirkenden Agens um eine protonierte, organische Säure handeln. In diesem Zusammenhang bevorzugte, protonierte, organische Säuren sind Anissäure, Benzoesäure, Ameisensäure, Valeriansäure, Zitronensäure, Glyoxylsäure, Glykolsäure, Glycerinphosphorsäure, Glutarsäure, Chloressigsäure, Chlorpropionsäure, Zimtsäure, Bernsteinsäure, Essigsäure, Weinsäure, Milchsäure, Brenztraubensäure, Fumarsäure, Propionsäure, 3-Hydroxypropionsäure, Malonsäure, Maleinsäure, Buttersäure, Isobuttersäure, Imidinoessigsäure, Apfelsäure, Isethionsäure, Methylmaleinsäure, Adipinsäure, Itaconsäure, Crotonsäure, Oxalsäure, Salizylsäure, Gallussäure, Sorbinsäure, Gluconsäure, Fettsäuren, insbesondere Stearinsäure und Adipinsäure und p-Oxybenzoesäure, wobei Zitronensäure am meisten bevorzugt ist.

- Als modifizierend wirkenden Agens kommen weiterhin Verbindungen umfassend mindestens ein Anion und ein Polykation in Betracht, in denen das Polykation die Struktur I

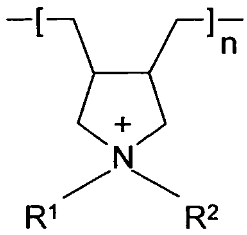

Struktur I

aufweist, in der

| | |
|---|---|
| $R^1$ und $R^2$ | gleich oder verschieden sein können und ein Kohlenwasserstoffrest mit 1 bis 10 Kohlenstoffatomen, vorzugsweise 1 bis 7 Kohlenstoffatomen, besonders bevorzugt 1 bis 4 Kohlenstoffatomen, darüber hinaus bevorzugt 1 bis 2 Kohlenstoffatomen und am meisten bevorzugt eine Methylgruppe darstellen und |
| n | einen Wert von mindestens 25, vorzugsweise mindestens 31, noch mehr bevorzugt mindestens 100, darüber hinaus bevorzugt mindestens 1.000, noch mehr bevorzugt mindestens 2.000, darüber hinaus noch mehr bevorzugt mindestens 2.500, noch mehr bevorzugt mindestens 3.000 und am meisten bevorzugt mindestens 5.000 hat. |

Besonders bevorzugte Polykationen der Struktur I sind Polydiallyldimethylamine. Bevorzugte Anionen sind das Chlorid-Ion, das Bromid-Ion, das Iodid-Ion, das Sulfid-Ion, das Sulfat-Ion, das Sulfit-Ion, das Nitrat-Ion, das Nitrit-Ion, das Acetat-Ion, das Lactat-Ion, das Carbonat-Ion, das Hydrogencarbonat-Ion, das Phosphat-Ion, das Phosphit-Ion und das Hydroxid-Ion. Neben diesen mono- bzw. divalenten Anionen kann die Verbindung auch polyvalente Anionen umfassen. Eine besonders bevorzugte Verbindung beinhaltend mindestens ein Anion und ein Polykation ist ein Polymer aus Diallyldimethylammoniumchlorid mit einem Wert für n von mindestens 25, vorzugsweise mindestens 31, darüber hinaus bevorzugt mindestens 100, darüber hinaus noch mehr bevorzugt mindestens 1.000, weiter bevorzugt mindestens 2.500 und am meisten bevorzugt mindestens 5.000. Besonders bevorzugt fallen unter die vorliegende Gruppe Verbindungen, die auf Polyallylaminen und auf Polyvinylaminen basieren.

- Auch kann es sich bei dem modifizierend wirkenden Agens um anorganische oder organische Feinteilchen handeln, wobei in diesem Fall das modifizierend wirkende Agens vorzugsweise in Form einer Dispersion eingesetzt wird. In einer anderen erfindungsgemäßen Ausgestaltung werden die Feinteilchen als solche, meist in Form einer separaten Zugabe eingesetzt. Es kann in diesem Fall vor oder auch nach dieser Zugabe der Feinteilchen oder vor und nach dieser Zugabe noch ein Binder eingesetzt werden, wozu sich beispielsweise ein oder mehrer der in den vor- und nachstehend aufgeführten Agens-Gruppen eignen. Bevorzugte organische oder anorganische Feinteilchen sind diejenigen Feinteilchen, die in der WO 2005/011860 A1 als bevorzugte organische bzw. anorganische Feinteilchen genannt werden. Besonders bevorzugt als anorganische Feinteilchen sind SiO-Verbindungen, insbesondere pyrogene Kieselsäure, wie sie beispielsweise unter der Handelbezeichnung Aerosil® erhältlich ist, Fällungskieselsäuren, wie sie kommerziell unter der Bezeichnung Sipernat® erhältlich sind, oder Kieselsäuresol, wie es beispielsweise unter der Handelsbezeichnung Levasil® erhältlich ist. Bevorzugte organische Feinteilchen sind auf Polysacchariden basierende Pulver, beispielsweise Zellulose- oder Zellulosederivat-Partikel, Stärke- oder Stärkederivat-Partikel oder Cyclodextrin-Partikel.

- Als weiteres modifizierend wirkendes Agens kommen wasserlösliche Polymere in Betracht. Bevorzugte wasserlösliche Polymer sind insbesondere Polyvinylalkohole oder wasserlösliche Polyethylenglykole. Das Molekulargewicht dieser wasserlöslichen Polymere liegt vorzugsweise in einem Bereich von 200 bis 100.000 g/mol, besonders bevorzugt in einem Bereich von 300 bis 50.000 g/mol und am meisten bevorzugt in einem Bereich von 350 bis 350.000 g/mol,

- Als modifizierend wirkendes Agens kommen, neben einer oder mehreren die Geruchsbildung verringernden Substanzen, wie sie unter anderem in "New Approach to Odor and pH Control in Personal Care", Jacek K. Dutkiewicz, Insight 2006, inernational Conference October 29 - November 2, 2006 und in US 2006/0029567 A1 beschrieben sind und einen Teil der vorliegenden Offenbarung darstellen, auch Verbindungen der Struktur II

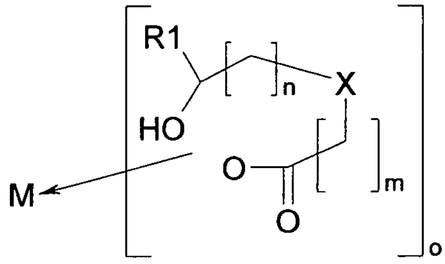

Struktur II

in Betracht, mit

R1 als einen $C_1$-$C_{20}$-, vorzugsweise einen $C_1$-$C_{15}$- sowie darüber hinaus bevorzugt einen $C_3$-$C_{10}$-Kohlenwasserstoff;

X als nicht vorhanden (in diesem Fall besteht eine direkte Kohlenstoff-Kohlenstoffbindung zwischen dem Kohlenstoff in $[]_n$ und dem Kohlenstoff in $[]_m$) oder ein in einem Bereich von 1 bis 5, vorzugsweise in einem Bereich von 1 bis 3 Doppelbindungen oder besonders bevorzugt eine einzige Doppelbindung aufweisendes, im Falle von mindestens 2 Doppelbindungen, vorzugsweise konjugiertes Doppelbindungssystem;

M als ein geladenes oder ungeladenes, vorzugsweise geladenes Metall;

m in einem Bereich von 1 bis 15, vorzugsweise in einem Bereich von 3 bis 12 und darüber hinaus bevorzugt in einem Bereich von 5 bis 9;

n in einem Bereich von 1 bis 5, vorzugsweise in einem Bereich von 1 bis 3 und darüber hinaus bevorzugt 1;

o in einem Bereich von 1 bis 4, vorzugsweise in einem Bereich von 1 bis 3 und besonders bevorzugt in einem Bereich von 1 bis 2 sowie darüber hinaus bevorzugt 2.

Diese Verbindungen der Struktur II können dabei vorteilhafterweise in Kombination mit einer Aminosäure, vorzugsweise mit Methionin, Arginin und Cystein, besonders bevorzugt mit Cystein oder Arginin, als modifizierend wirkendes Agens eingesetzt werden.

- Weiterhin kommen auch antimikrobielle Verbindungen als Mofizierungsmittel in Betracht, wobei insbesondere Silber enthaltene Verbindungen als antimikrobielle Verbindungen besonders bevorzugt sind. Bevorzugt Silber enthaltende Verbindungen sind beispielsweise kolloidales Silber, schwerlösliche Silbersalze, wie beispielsweise Silberchlorid, Silberbromid oder Silberiodid, oder aber wasserlösliche Silbersalze, wie beispielsweise Silbernitrat. Erfindungsgemäß besonders bevorzugte, Silber enthaltende Verbindungen sind diejenigen Silbersalze, die in der WO-A-03/090799 als bevorzugte wasserlösliche Silbersalze genannt werden. Als Silbersalz insbesondere bevorzugt sind Silbersalze ausgewählt aus der Gruppe bestehend aus Silberacetat, Silberacetylacetonat, Silberazid, Silberacetylid, Silberarsenat, Silberbenzoat, Silberbifluorid, Silbermonofluorid, Silberfluorid, Silber borfluorid, Silberbromat, Silberbromid, Silbercarbonat, Silberchlorid, Silberchlorat, Silberchromat, Silbercitrat, Silbercyanat, Silbercyanid, Silber-(cis,cis-1,5-cyclooctadiene)-1,1,1,5,5,5,hexafluoroacetylacetonat, Silberdichromat-tetrakis-(pyridin)-Komplex, Silberdiethyldithiocarbamat, Silber(I)fluorid, Silber(II)fluorid, Silber-7,7-dimethyl1,1,1,2,2,3,3,-heptafluor-4,6-octandionat, Silberhexafluoroantimonat, Silberhexafluoroarsenat, Silberhexafluorophosphat, Silberiodat, Silberiodid, Silberisothiocyanat, Silberkaliumcyanid, Silberlactat, Silbermolybdat, Silbernitrat, Silbernitrit, Silber(I)oxid, Silber(II)oxid, Silberoxalat, Silberperchlorat, Silberperfluorobutyrat, Silberperfluoropropionat, Silberpermanganat, Silberperrhenat, Silberphosphat, Silberpicratmonohydrat, Silberpropionat, Silberselenat, Silberselenid, Silberselenit, Silbersulfadiazin, Silbersulfat, Silbersulfid, Silbersulfit, Silbertellurid, Silbertetrafluoroborat, Silbertetraiodomecurat, Silbertetratungstenat, Silberthiocyanat, Silber-p-toluensulfonat, Trifluoromethanesulfonsäure-Silbersalz, Trifluoroessigsäure-Silbersalz und Silbervanadat. Auch Mischungen aus mindestens zwei der vorstehend genannten Silbersalze können eingesetzt werden. Am meisten bevorzugt sind Silberacetat und Silbernitrat.
Im Zusammenhang mit dem vorstehend beschriebenen Einsatz von Silber enthaltenden Verbindungen als Modifizierungsmittel ist es insbesondere bevorzugt, dass diese Silber enthaltenden Verbindungen einem bereits oberflächennachvernetzten, wasserabsorbierenden Polymergebilde zugesetzt werden.

- Auch reines Wasser kann als Modifizierungsmittel eingesetzt werden, etwa zur Nachbefeuchtung der wasserabsorbierenden Polymergebilde.

- Ferner können zwei, drei, vier oder mehr der vorstehend aufgeführten Varianten eines modifizierenden Agens bzw. Modifizierungsmittels in Kombination miteinander eingesetzt werden.

Erfindungsgemäß besonders bevorzugte Modifizierungsmittel, die insbesondere als Oberflächenmodifizierungsmittel eingesetzt werden können, sind eines, zwei oder mehr ausgewählt aus der Gruppe bestehend aus

i) einer wässrigen Lösung umfassend Wasser und mindestens einen in Wasser gelösten Oberflächennachvernetzer;
ii) einer wässrigen Lösung umfassend Wasser und mindestens ein in Wasser gelöstes, wasserlösliches Polymer, besonders bevorzugt ein Polyethylenglykol, insbesondere mit einem Molekulargewicht in einem Bereich von 300 bis 15.000 g/mol;
iii) einer wässrigen Suspension umfassend Wasser, eine protonierte, organische Säure, vorzugsweise Zitronensäure, sowie ein anorganisches Feinteilchen, vorzugsweise eine SiO-Verbindung, besonders bevorzugt eine Fällungskieselsäure;
iv) einer wässrigen Lösung umfassend Wasser sowie mindestens ein organisches Metallsalz, vorzugsweise Aluminiumlactat;
v) einer wässrigen Lösung umfassend Wasser, mindestens einen in Wasser gelösten Oberflächennachvernetzer sowie ein organisches Metallsalz, vorzugsweise Aluminiumlactat;
vi) einer wässrigen Lösung umfassend Wasser sowie ein anorganisches Metallsalz, vorzugsweise Aluminiumsulfat;
vii) einer wässrigen Lösung umfassend Wasser, mindestens einen Oberflächennachvernetzer sowie ein anorganisches Metallsalz, vorzugsweise Aluminiumsulfat;
viii) einem anorganischen Pulver, vorzugsweise ein anorganisches Metallsalz, besonders bevorzugt Aluminiumsulfat, Aluminiumchlorid oder Aluminiumphosphat, oder eine SiO-Verbindung, vorzugsweise eine pyrogene Kieselsäure oder eine Fällungskieselsäure;
ix) einem organischen Pulver, vorzugsweise auf Polysacchariden basierende Pulver, beispielsweise Carboxyzellulosepartikel, oder Cyclodextrin-Partikel;
x) einer wässrigen Dispersion umfassend Wasser, mindestens ein anorganisches oder organisches Metallsalz, vorzugsweise Aluminiumsulfat oder Aluminiumlactat, sowie mindestens ein Oxid eines Metalls, vorzugsweise ein Zinkoxid;
xi) einer wässrigen Dispersion umfassend Wasser, mindestens ein Oberflächennachvernetzter, mindestens ein anorganisches oder organisches Metallsalz, vorzugsweise Aluminiumsulfat oder Aluminiumlactat, sowie mindestens ein Oxid eines Metalls, vorzugsweise ein Zinkoxid;
xii) einer wässrigen Lösung umfassend Wasser und mindestens ein Polykation, vorzugsweise ein Polydiallyldimethylammoniumchlorid;
xiii) einer wässrigen Lösung umfassend Wasser, mindestens einen Oberflächennachvernetzer und mindestens ein Polykation, vorzugsweise ein Polydiallyldimethylammoniumchlorid;
xiv) einer wässrigen Dispersion umfassend Wasser und ein in Wasser dispergiertes, thermoplastisches Polymer;
xv) einer wässrigen Dispersion umfassend Wasser, mindestens einen Oberflächennachvemetzer und ein in Wasser dispergiertes, thermoplastisches Polymer;
xvi) einer wässrigen Lösung bzw. einer wässrigen Dispersion umfassend eine Verbindung der Struktur II, vorzugsweise ein unter der Handelsbezeichnung "Tegosorb®" erhältliches Produkt der Goldschmidt GmbH, Wasser sowie gegebenenfalls ein anorganisches Feinteilchen, vorzugsweise eine SiO-Verbindung, besonders bevorzugt eine Fällungskieselsäure;
xvii) Wasser, insbesondere destilliertes Wasser, mittel Ionenaustauscher entionisiertes Wasser oder aber Leitungswasser;
xviii) wässrigen Lösungen oder wässrigen Suspensionen basierend auf organischen Feststoffen, vorzugsweise auf Polysacchariden basierende Pulver, beispielsweise Carboxyzellulosepartikel, oder Cyclodextrin-Partikel;
xix) einer wässrigen Lösung eines wasserlöslichen Silbersalzes;
xx) einer wässrigen Dispersion einer wasserunlöslichen Silbersalzes.

Es ist weiterhin erfindungsgemäß bevorzugt, dass das modifizierend wirkende Agens mit dem wasserabsorbierenden Polymergebilde im Verfahrensschritt b) in einer Menge in einem Bereich von 0,001 bis 20 Gew.-%, besonders bevorzugt 0,001 bis 10 Gew.-% und am meisten bevorzugt 0,01 bis 5 Gew.-%, bezogen auf das Gewicht des wasserabsorbierenden Polymergebildes, in Kontakt gebracht wird, wobei die Menge an eingesetztem, modifizierend wirkenden

Agens entscheidend von der Art des Agens abhängig ist. Bei einem Einsatz von Silber enthaltenden Verbindungen ist es bevorzugt, dass diese in einer solchen Menge eingesetzt werden, dass 0,1 bis 1.000, bevorzugt 1 bis 500 ppm und am meisten bevorzugt 10 bis 100 ppm Silber, bezogen auf das Gewicht des wasserabsorbierenden Polymergebildes, zum Einsatz gebracht werden. Weiterhin ist es bevorzugt, dass das wasserabsorbierende Polymergebilde im Verfahrensschritt b) mit Wasser in einer Menge in einem Bereich von 0,5 bis 30 Gew.-%, besonders bevorzugt 1 bis 20 Gew.-% und am meisten bevorzugt 1,5 bis 10 Gew.-%, jeweils bezogen auf das Gewicht der wasserabsorbierenden Polymergebilde, in Kontakt gebracht wird.

Das in Kontakt bringen des Modifizierungsmittels mit den wasserabsorbierenden Polymergebilden im Verfahrensschritt b) erfolgt vorzugsweise durch Vermischen in dem Fachmann bekannten Mischvorrichtungen, vorzugsweise in einem Patterson-Kelley-Mischer, einem DRAIS-Turbulenzmischer, einem Lödigemischer, einem Ruberg-Mischer, einem Schneckenmischer, einem Tellermischer, einem Wirbelschichtmischer oder aber in einem kontinuierlich arbeitenden senkrechten Mischer, in denen das Polymergebilde mittels rotierender Messer in schneller Frequenz gemischt wird (Schugi-Mischer). Die durchschnittliche Verweilzeit der mit dem Modifizierungsmittel in Kontakt gebrachten, wasserabsorbierenden Polymergebilden in diesen Mischern liegt vorzugsweise in einem Bereich von 0,01 bis 240 Sekunden, besonders bevorzugt in einem Bereich von 0,05 bis 180 Sekunden und am meisten bevorzugt in einem Bereich von 0,1 bis 120 Sekunden.

Im Verfahrensschritt c) des erfindungsgemäßen Verfahrens wird nun das mit dem Modifizierungsmittel in Kontakt gebrachte, wasserabsorbierende Polymergebilde weiterbehandelt, wobei die Weiterbehandlung im Verfahrensschritt c) zumindest teilweise in einem sich drehenden Behälter erfolgt. Bei dem Weiterbehandeln im Verfahrensschritt c) handelt es sich vorzugsweise um ein Mischen des mit dem Modifizierungsmittel in Kontakt gebrachten, wasserabsorbierenden Polymergebildes.

Gemäß einer bevorzugten Ausführungsform des erfindungsgemäßen Verfahrens sind die im Verfahrensschritt c) eingesetzten, mit dem Modifizierungsmittel in Kontakt gebrachten, wasserabsorbierenden Polymergebilde durch einen nach ERT 430.2-02 bestimmten Feuchtigkeitsgehalt von höchstens 30 Gew.-%, bevorzugt höchstens 25 Gew.-%, noch mehr bevorzugt höchstens 20 Gew.-% und am meisten bevorzugt höchstens 15 Gew.-%, jeweils bezogen auf das Gesamtgewicht des wasserabsorbierenden Polymergebildes, gekennzeichnet.

Sofern das in Kontakt bringen des wasserabsorbierenden Polymergebildes mit dem Modifizierungsmittel im Verfahrensschritt b) nicht bereits in einem sich drehenden Behälter erfolgte, so wird das mit dem Modifizierungsmittel in Kontakt gebrachte, wasserabsorbierende Polymergebilde zunächst in einen sich drehenden Behälter überführt.

Der sich drehende Behälter umfasst vorzugsweise einen horizontal gelagerten, trommelartig ausgebildeten Behälter, der mittels geeigneter Motoren in eine Drehung um eine horizontal durch die Mitte des Behälters verlaufende Achse versetzt werden kann. Der Behälter umfasst entlang dieser Achse einen hinteren Kopfbereich, einen vorderen Kopfbereich und einen den hinteren und vorderen Kopfbereich verbindenden Mantelbereich. Grundsätzlich kann der Behälter aus Kunststoff oder auch aus Stahl gefertigt sein, vorzugsweise jedoch ist der Behälter aus Edelstahl gefertigt, wobei die Innenflächen des Behälters, die mit dem wasserabsorbierenden Polymergebilde in Kontakt treten können, gegebenenfalls mit geeigneten Polymeren, beispielsweise mit Polypropylen- oder Poly(tetrafluorethylen)-Polymeren, beschichtet sein kann. Der Behälter ist vorzugsweise zylinderartig ausgestaltet, wobei der hintere und der vordere Kopfbereich gegebenenfalls haubenartig ausgebildet sein können. Unter die zylinderartige Ausgestaltung fallen auch solche, in denen der Mantelbereich nicht streng zylindrisch ausgestaltet ist, sondern beispielsweise eine bauchige oder gewölbte Form oder stundenglasähnlich verengt ausgestaltet ist.

Der Durchmesser des Behälters liegt vorzugsweise in einem Bereich von 1 Meter bis 15 Meter, besonders bevorzugt 2 Meter bis 10 Meter und am meisten bevorzugt 2,5 Meter bis 5 Meter liegt, während die Länge des Behälters vorzugsweise in einem Bereich von 1 Meter bis 20 Meter, besonders bevorzugt 2 Meter bis 10 Meter und am meisten bevorzugt 3 Meter bis 7 Meter liegt. Das Füllvolumen des sich drehenden Behälters liegt vorzugsweise in einem Bereich von 100 bis 250.000 Litern, besonders bevorzugt 1.000 bis 100.000 Litern und am meisten bevorzugt 5.000 bis 50.000 Litern.

An den Innenseiten des Behälters, die mit dem wasserabsorbierenden Polymergebilde in Berührung kommen können, sind vorzugsweise als Schaufeln ausgestaltete Mischhilfen angebracht, wobei diese ebenfalls aus Kunststoff oder Metall gefertigt sein können, vorzugsweise jedoch aus Edelstahl ausgebildet sind und gegebenenfalls auch mit geeigneten Polymeren, beispielsweise mit Polypropylen- oder Poly(tetrafluorethylen)-Polymeren, beschichtet sein können. Weiterhin ist es bevorzugt, dass die Mischhilfen fest an den Innenseiten des Behälters befestig sind, wobei eine Befestigung mit dem Mantelbereich des Behälters am meisten bevorzugt ist. Weiterhin ist es bevorzugt, dass die Mischhilfen schalenartig ausgebildet sind. Die Anzahl der Mischhilfen innerhalb des Behälters liegt vorzugsweise in einem Bereich von 1 bis 50, besonders bevorzugt 2 bis 25 und am meisten bevorzugt 3 bis 6.

Der Vorteil des Einsatzes eines solchen, sich drehenden Behälters besteht beispielsweise darin, dass der gesamte Inhalt des Behälters jederzeit bewegt und durch die Mischhilfen umgeschichtet wird, wobei das wasserabsorbierende Polymergebilde innerhalb des Behälters entlang einer schlaufenförmigen Bahn bewegt wird. Da sich im gesamten Behälter, abgesehen von einer möglicherweise beweglichen Ein- und Auslasseinrichtung, vorzugsweise keine

beweglichen oder gegenläufigen Teile befinden, unterliegt das wasserabsorbierende Polymergebilde während des Drehens des Behälters, wenn überhaupt, nur einer geringen Friktion. Ein Mahleffekt, welcher oftmals in den herkömmlich eingesetzten Konusmischem oder in anderen Stator-Rotor-Mischern beobachtet wird, tritt wenn überhaupt nur sehr vermindert auf.

Weiterhin beinhaltet der sich drehende Behälter nach einer weiteren erfindungsgemäßen Ausgestaltung im vorderen Kopfbereich einen Produkteinlass, der optional mit Einlasshilfen wie Schnecken, Schiebern, Rüttlern, Zellenradschläusen oder Druckluftquellen oder einer Kombination aus mindestens zwei davon ausgestattet sein kann, über den das mit dem Modifizierungsmittel in Kontakt gebrachte, wasserabsorbierende Polymergebilde in den sich drehenden Behälter eingebracht werden kann und einen vorzugsweise regelbaren Produktauslass, über den dass im Behälter behandelte wasserabsorbierende Polymergebilde aus dem sich drehenden Behälter ausgetragen werden kann. Über geeignete Elektromotoren kann der Behälter in Drehung versetzt werden, wobei die Umdrehungsgeschwindigkeit vorzugsweise in einem Bereich 1 bis 100 Umdrehungen pro Minute, besonders bevorzugt 2 bis 50 Umdrehungen pro Minute und am meisten bevorzugt 3 bis 15 Umdrehungen pro Minute liegt.

Ferner beinhaltet der sich drehende Behälter als Auslasseinheit vorzugsweise eine Lanze, die vorzugsweise durch die Mitte des hinteren Kopfbereiches entlang der Rotationsachse des sich drehenden Behälters eingeführt werden kann, wobei diese Lanze ebenfalls aus Kunststoff oder Metall gefertigt werden kann, vorzugsweise jedoch aus Edelstahl ausgebildet ist. Weiterhin ist es bevorzugt, dass die Lanze nicht fest mit dem drehbaren Behälter verbunden ist, dass sich also die Lanze nicht um ihre Längsachse dreht, wenn auch der Behälter in Drehung versetzt wird. An der Lanze kann, im Inneren des Behälters, ein Austragselement angebracht sein, in dem vorzugsweise eine Vertiefung zur Aufnahme bzw. Führung von Stoffen ausgebildet ist, über welches das wasserabsorbierende Polymergebilde in Richtung des Auslasses geleitet wird. Durch die Befestigung dieses Austragselementes an der bewegbaren Lanze ist auch das Austragselement bewegbar im Inneren des Behälters angeordnet.

Des Weiteren kann der sich drehende Behälter als eine der Einlasseinheiten eine Zuführung für ein Modifizierungsmittel umfassen, wobei diese Zuführung vorzugsweise im vorderen Kopfbereich angebracht ist. Bei dieser Zuführung handelt es sich vorzugsweise um eine im Inneren des sich drehenden Behälters vorzugsweise am vorderen oftmals statischen Kopfbereich, vorzugsweise fixiert, angeordnete Sprühdüse, über die insbesondere Flüssigkeiten, beispielsweise Lösungen, Dispersionen oder Emulsion, aber gegebenenfalls auch Feststoffe, wie etwa Pulver, auf das wasserabsorbierende Polymergebilde appliziert werden können.

Gemäß einer besonders bevorzugten Ausführungsform des erfindungsgemäßen Verfahrens wird im Verfahrensschritt c) eine Mischvorrichtung der Firma Lindor, Dordrecht, Niederlande eingesetzt, wobei diese Mischvorrichtung vorzugsweise eine Mischvorrichtung ausgewählt aus der Gruppe bestehend aus den Mischvorrichtungen Lindor 70, Lindor 200, Lindor 500, Lindor 750, Lindor 1000, Lindor 1500, Lindor 2000, Lindor 2300, Lindor 4000, Lindor 7000, Lindor 8000, Lindor 12000, Lindor 14000 und Lindor 25000 ist. Die vorstehenden genannten Typen können bezüglich ihrer Ein- und Auslasseinrichtungen für einen kontinuierlichen Betrieb ausgelegt sein. Neben einer An- und Abfahrphase liegt ein kontinuierlicher betrieb erfindungsgemäß vorzugsweise dann vor, wenn über eine Zeitraum von mindestens 1, vorzugsweise mindestens 2 und besonders bevorzugt mindestens 10 Stunden nach der meist als Befüllen erfolgenden Anfahrphase ein ununterbrochener Ein- und Austrag von aus dem Behälter erfolgt.

Gemäß einer weiteren, besonderen Ausführungsform des erfindungsgemäßen Verfahrens kann der im Verfahrensschritt c) eingesetzte, sich drehende Behälter eine Heiz- oder Kühlvorrichtung umfassen, welche es ermöglicht, das sich im drehenden Behälter befindliche, wasserabsorbierende Polymergebilde zumindest teilweise zu erhitzen oder aber abzukühlen. Dabei kann es vorteilhaft sein, wenn diese Heiz- oder Kühlvorrichtung zumindest einen Teil des Mantelbereiches des sich drehenden Behälters erhitzt bzw. kühlt und der so erhitzte bzw. gekühlte Mantelbereich dann die Wärme an das wasserabsorbierende Polymergebilde weitergibt bzw. Wärme des wasserabsorbierenden Polymergebildes aufnimmt. Im Falle einer Heizvorrichtung ist insbesondere auch der Einsatz von Wärmequellen, beispielsweise von Infrarotlampen, im Inneren des sich drehenden Behälters oder aber der Eintrag von heißen Gasen, wie beispielsweise heißer Luft, möglich. Dabei kann es insbesondere vorteilhaft sein, dass Erhitzen oder aber das Kühlen der wasserabsorbierenden Polymergebilde auf einen definierten Bereich zwischen dem vorderen und dem hinteren Kopfbereich des sich drehenden Behälters zu beschränken.

Die durchschnittliche Verweilzeit (sie wird berechnet aus dem Quotienten des Volumens des Drehbaren Behälters zum zugeführten Volumenstrom) der wasserabsorbierenden Polymergebilde in dem sich drehenden Behälter liegt vorzugsweise in einem Bereich von 5 bis 300 Minuten, besonders bevorzugt in einem Bereich von 10 bis 180 Minuten und am meisten bevorzugt in einem Bereich von 15 bis 140 Minuten. Auch ist es bevorzugt, dass im Verfahrensschritt c) der Behälter bis maximal 80 % seiner größten Querschnittsfläche (gemeint ist diejenige Querschnittsfläche, die Senkrecht von der Rotationsachse durchstoßen wird), besonders bevorzugt maximal 70 %, noch mehr bevorzugt maximal 60 % und am meisten bevorzugt maximal 50 % sowie weiterhin bevorzugt maximal 40 % befüllt ist. So ist in der Regel maximal 60 Vol.-%, vorzugsweise maximal 55 Vol.-%, bevorzugt maximal 50 Vol.-% und darüber hinaus bevorzugt 40 Vol.-% des Innvolumen des Behälters gefüllt

Gemäß einer besonders bevorzugten Ausführungsform des erfindungsgemäßen Verfahrens erfolgt das Weiterbehandeln im Verfahrensschritt c) zumindest teilsweise kontinuierlich. Dies wird beispielsweise dadurch realisiert, dass das mit dem Modifizierungsmittel in Kontakt gebrachte, wasserabsorbierende Polymergebilde über den Einlass im vorderen Kopfbereich des sich drehenden Behälters eingetragen wird, den sich drehenden Behälter durchströmt und dann, nach einer bestimmten Verweilzeit, den sich drehenden Behälter über den Auslass am hinteren Kopfbereich verlässt. Die durchschnittliche Verweilzeit eines wasserabsorbierenden Polymerpartikels ist dabei unter anderem von der pro Zeiteinheit über den Einlass zugeführten Menge an mit dem Modifizierungsmittel in Kontakt gebrachtem, wasserabsorbierendem Polymergebilde, vom Volumen des sich drehenden Behälters und von der über den Auslass pro Zeiteinheit austretenden Menge an behandeltem, wasserabsorbierenden Polymergebilde abhängig. Der Vorteil eines kontinuierlichen Betriebes eines sich drehenden Mischers zur Behandlung des in der Regel mit einer vergleichsweise kleinen Menge Modifizierungsmittel in Kontakt gebrachten vergleichsweise großen Menge wasserabsorbierenden Polymergebilde liegt insbesondere darin, dass es zu einer sehr raschen Verteilung des Modifizierungsmittels in dem Wasserabsorbierenden Polymergebilde kommt. Dieses ist insbesondere der Fall, wenn das wasserabsorbierende Polymergebilde oder auch die Mischung aus Modifizierungsmittel und wasserabsorbierenden Polymergebilde erdfeucht ist und daher zum anhaften bzw. zur Klumpenbildung neigt. Die Regulierung der den Behälter strömenden Mengen kann neben einer einstellbaren Einlasseinrichtung insbesondere durch eine einstellbare Auslasseinrichtung ermöglicht werden. Diese Auslasseinrichtung weist vorzugsweise eine verstellbare Auslassöffnung auf. Als Auslasseinrichtungen kommen neben einem Wehr, vorzugsweise einem vertikal regalbern Wehr, auch Zellradschleusen, Austragschnecken, die insbesondere Frequenzgeregelt sein können, oder mindestens zwei davon in Betracht.

Insbesondere dann, wenn das Behandeln im Verfahrensschritt c) kontinuierlich erfolgt, hat es sich auch als vorteilhaft erwiesen, wenn im Bereich des Auslasses am hinteren Kopfbereich eine Auslasseinrichtung mit einer variablen Auslassöffnung vorgesehen ist, die vorzugsweise wehrartig ausgestaltetet ist. Hierbei ist es bevorzugt, ein Wehr in der Auslassöffnung auszubilden, dessen Querschnittsfläche der Querschnittsfläche des Auslasses entspricht, wobei die Querschnittsfläche des Wehrs mechanisch oder auch elektromechanisch verkleinert werden kann. Entspricht die Querschnittsfläche des Wehrs der Querschnittsfläche des Auslasses, so ist der Auslass verschlossen und das wasserabsorbierende Polymergebilde kann den sich drehenden Behälter nicht verlassen. Wird nun die Querschnittsfläche des Wehr verkleinert, beispielsweise in dem im Falle eines kreisförmigen Wehrs, welches in einem kreisförmigen Auslass angebracht ist, die obere Kreishälfte um eine durch die Mitte des kreisförmigen Wehrs verlaufende Achse gedreht wird, so kann das wasserabsorbierende Polymergebilde durch den so entstehenden Spalt den sich drehenden Behälter verlassen. Der Auslas ist daher über das Wehr regelbar. Über die Regelung der Querschnittsfläche lässt sich insbesondere bei einem kontinuierlichen Behandeln des wasserabsorbierenden Polymergebildes im Verfahrensschritt c) die durchschnittliche Verweilzeit der wasserabsorbierenden Polymergebilde in dem sich drehenden Behälter variieren.

Weiterhin ist es gemäß einer besonderen Ausgestaltung des erfindungsgemäßen Verfahrens auch denkbar, dass das in Kontakt bringen des wasserabsorbierenden Polymergebildes mit dem Modifizierungsmittel im Verfahrensschritt b) zumindest teilweise ebenfalls in einem sich drehenden Behälter erfolgt. In diesem Fall werden die Verfahrensschritte b) und c) zumindest teilweise gleichzeitig ausgeführt, in dem ein gegebenenfalls noch überhaupt nicht mit einem Modifizierungsmittel in Kontakt gebrachtes, wasserabsorbierendes Polymergebilde oder aber ein bereits teilweise mit einem Modifizierungsmittel in Kontakt gebrachtes, wasserabsorbierendes Polymergebilde über den Einlass in den sich drehenden Behälter eingetragen wird, durch eine Zuführung im Inneren des Behälters, vorzugsweise im Bereich des vorderen Kopfbereiches, das wasserabsorbierende Polymergebilde dann mit dem Modifizierungsmittel vorzugsweise mittels einer Düse besprüht wird und das mit dem Modifizierungsmittel besprühte, wasserabsorbierende Polymergebilde anschließend in dem sich drehenden Behälter durch Vermischen weiterbehandelt wird.

Erfindungsgemäß besonders bevorzugte Ausführungsformen des Verfahrens zur Herstellung eines Superabsorbers sind ausgewählt aus den folgenden Verfahren:

($\gamma$1) Ein mit weniger als 25 Gew.-%, vorzugsweise weniger als 10 und besonders bevorzugt weniger als 5 Gew.-%, jeweils bezogen auf das oder die eingesetzten Modifikationsmittel, weiterhin vorzugsweise noch überhaupt nicht, mit einem Modifizierungsmittel in Kontakt gebrachtes, wasserabsorbierendes Polymergebilde wird in den sich drehenden Mischer eingebracht, dort vorzugsweise im vorderen Kopfbereich mit einem Modifizierungsmittel in Kontakt gebracht und dann in dem sich drehenden Behälter durch Mischen weiterbehandelt, wobei insbesondere in den Fällen, in denen das Modifizierungsmittel einen Nachvernetzer beinhaltet, das wasserabsorbierende Polymergebilde im Inneren des sich drehenden Behälters noch erhitzt werden kann. Als Modifizierungsmittel bei dieser Ausführungsform des erfindungsgemäßen Verfahrens kommen insbesondere alle vorstehend genannten Modifizierungsmittel i) bis xvii) für sich oder als Kombination von mindestens zwei davon in Betracht.

($\gamma$2) Ein Verfahren gemäß ($\gamma$1), wobei jedoch ein bereits oberflächennachvernetztes, wasserabsorbierendes Polymergebilde eingesetzt wird. Als Modifizierungsmittel bei dieser Ausführungsform des erfindungsgemäßen Verfahrens kommen insbesondere die vorstehend genannten Modifizierungsmittel ii), iii), iv), vi) viii), ix), x) xii), xiv), xvi), xvii), xix) und xx) für sich oder als Kombination von mindestens zwei davon in Betracht.

($\gamma$3) Ein mit weniger als 25 Gew.-%, vorzugsweise weniger als 10 und besonders bevorzugt weniger als 5 Gew.-%,

jeweils bezogen auf das oder die eingesetzten Modifikationsmittel, weiterhin vorzugsweise noch überhaupt nicht mit einem Modifizierungsmittel in Kontakt gebrachtes, wasserabsorbierendes Polymergebilde wird, bevor es in den sich drehenden Mischer eingebracht wird, mit einem Modifizierungsmittel durch Vermischen in Kontakt gebracht. Als Modifizierungsmittel bei dieser Ausführungsform des erfindungsgemäßen Verfahrens kommen insbesondere die vorstehend genannten Modifizierungsmittel i) bis xvii) für sich oder als Kombination von mindestens zwei davon in Betracht.

(γ4) Ein Verfahren gemäß (γ3), wobei jedoch ein bereits oberflächennachvernetztes, wasserabsorbierendes Polymergebilde eingesetzt wird. Als Modifizierungsmittel bei dieser Ausführungsform des erfindungsgemäßen Verfahrens kommen insbesondere die vorstehend genannten Modifizierungsmittel ii), iii), iv), vi) viii), ix), x) xii), xiv), xvi), xvii), xix) und xx) für sich oder als Kombination von mindestens zwei davon in Betracht.

(γ5) Ein noch überhaupt nicht mit einem Modifizierungsmittel in Kontakt gebrachtes, wasserabsorbierendes Polymergebilde wird in den sich drehenden Mischer eingebracht, dort vorzugsweise im vorderen Kopfbereich mit einem ersten Modifizierungsmittel in Kontakt gebracht und dann in dem sich drehenden Behälter durch Mischen weiterbehandelt, wobei insbesondere in den Fällen, in denen das erste Modifizierungsmittel einen Nachvernetzer beinhaltet, das wasserabsorbierende Polymergebilde im Inneren des sich drehenden Behälters noch erhitzt werden kann. In einem stromabwärts von der Zugabe des ersten Modifizierungsmittels gelegenen Bereich wird das wasserabsorbierende Polymergebilde dann mit einem zweiten Modifizierungsmittel in Kontakt gebracht und dann erneut weiterbehandelt. Das in Kontakt bringen des wasserabsorbierenden Polymergebildes mit dem ersten und dem zweiten Modifizierungsmittel und das anschließende Weiterbehandeln können dabei in ein und demselben, sich drehenden Behälter erfolgen. Denkbar ist aber auch, zwei oder mehr hintereinander geschaltete, sich drehende Behälter einzusetzen. Als erstes Modifizierungsmittel bei dieser Ausführungsform des erfindungsgemäßen Verfahrens kommen insbesondere alle vorstehend genannten Modifizierungsmittel i) bis xvii) für sich oder als Kombination von mindestens zwei davon in Betracht, während als zweites Modifizierungsmittel vorzugsweise die Modifizierungsmittel ii), iii), iv), vi) viii), ix), x) xii), xiv), xvi), xvii), xix) oder xx) für sich oder als Kombination von mindestens zwei davon eingesetzt werden können.

(γ6) Ein Verfahren gemäß (γ5), wobei jedoch ein bereits oberflächennachvernetztes, wasserabsorbierendes Polymergebilde eingesetzt wird. Als erstes und als zweites Modifizierungsmittel bei dieser Ausführungsform des erfindungsgemäßen Verfahrens kommen insbesondere die vorstehend genannten Modifizierungsmittel ii), iii), iv), vi) viii), ix), x) xii), xiv), xvi), xix) oder xx) für sich oder als Kombination von mindestens zwei davon in Betracht.

Die erfindungsgemäßen Superabsorber sind vorzugsweise durch einen gemäß der hierin beschriebenen Testmethode bestimmten Staubanteil von höchstens 8, besonders bevorzugt höchstens 6, darüber hinaus noch mehr bevorzugt von höchstens 4 und am meisten bevorzugt von höchstens 2 gekennzeichnet.

Weiterhin ist es erfindungsgemäß bevorzugt, dass die erfindungsgemäßen Superabsorber einen gemäß ERT 430.2-02 bestimmten Feuchtigkeitsgehalt in einem Bereich von 2 bis 10 Gew.-%, besonders bevorzugt 2 bis 8 Gew.-%, noch mehr bevorzugt 2 bis 6 Gew.-% und am meisten bevorzugt 2 bis 5 Gew.-% aufweisen. Liegt der Feuchtigkeitsgehalt außerhalb des Bereiches von 2 bis 10 Gew.-%, so werden die Absorptions- und Fördereigenschaften des Superabsorbers nachteilig beeinflusst. Dabei kann der vorstehend angegebene Feuchtigkeitsgehalt des Superabsorbers demjenigen Feuchtigkeitsgehalt entsprechen, den der Superabsorber nach seiner Herstellung und insbesondere vor seiner Einarbeitung in einen Hygieneartikel aufweist. Der Feuchtigkeitsgehalt kann jedoch auch demjenigen Feuchtigkeitsgehalt entsprechen, den der Superabsorber aufweist, wenn er aus einem Hygieneartikel, beispielsweise einer Windel, isoliert wird. Da der Superabsorber, wenn er bereits in einen Hygieneartikel eingearbeitet wurde, bereits Wasser, welches in Form von Wasserdampf stets in der Umgebungsluft enthalten ist, absorbiert haben kann, ist es durchaus möglich, dass der Feuchtigkeitsgehalt eines sich in einem Hygieneartikel befindlichen Superabsorbers gegebenenfalls den oberen Grenzwert von 10 Gew.-% überschreitet.

Einen Beitrag zur Lösung mindestens einer der eingangs genannten Aufgaben leistet auch eine Vorrichtung zur Herstellung eines Superabsorbers, beinhaltend fluidleitend miteinander verbunden:

- einen Polymerisationsbereich; gefolgt von
- einem Trocknungsbereich; gefolgt von
- einem Weiterbehandlungsbereich;

wobei der Weiterbehandlungsbereich einen drehbaren Behälter beinhaltet.

Der Polymerisationsbereich umfasst vorzugsweise eine Polymerisationsvorrichtung, wobei als Polymerisationsvorrichtung alle dem Fachmann bekannten Vorrichtungen eingesetzt werden können, die sich zur Herstellung von auf Polyacrylaten basierenden Polymergelen einsetzten lassen, wobei kontinuierlich arbeitende Polymerisationsvorrichtungen, wie beispielsweise Polymerisationsbänder, besonders bevorzugt sind. Geeignete Polymerisationsvorrichtungen sind beispielsweise im Absatz 3.2.3 in "Modern Superabsorbent Polymer Technology" von F.L. Buchholz und A.T.

Graham, Wiley-VCH, 1998 beschrieben.

Der Trocknungsbereich umfasst vorzugsweise eine Trocknungsvorrichtung, wobei als Trocknungsvorrichtung ebenfalls alle dem Fachmann bekannten Trocknungsvorrichtungen eingesetzt werden können. Bevorzugte Trocknungsvorrichtungen umfassen insbesondere Drehrohröfen, Wirbelbetttrockner, Tellertrockner, Paddeltrockner und Infrarottrockner.

Weiterhin ist es vorteilhaft, wenn der Polymerisationsbereich noch einen Gelzerkleinerungsbereich umfassend eine Gelzerkleinerungsvorrichtung, wie etwa ein Fleischwolf oder ein Schneidmesser, umfasst, so dass das Gel vor der Trocknung noch zerkleinert werden kann. Auf diese Weise ist eine effizientere Trocknung des Polymergels möglich. Geeignete Gelzerkleinerungsvorrichtungen sind beispielsweise im Absatz 3.2.4 in "Modern Superabsorbent Polymer Technology" von F.L. Buchholz und A.T. Graham, Wiley-VCH, 1998 beschrieben. Auch kann es sich als vorteilhaft erweisen, wenn der Trocknungsbereich neben der Trocknungsvorrichtung noch eine Mahlvorrichtung und/oder eine Siebvorrichtung umfasst, so dass die wasserabsorbierenden Polymergebilde vor dem Eintritt in den Weiterbehandlungsbereich noch zerkleinert und/oder auf eine bestimmte Partikelgröße, vorzugsweise auf eine Partikelgröße in einem Bereich von 150 bis 850 $\mu$m, abgesiebt werden können. Geeignete Mahlvorrichtungen und Siebvorrichtungen sind beispielsweise im Absatz 3.2.6 in "Modern Superabsorbent Polymer Technology" von F.L. Buchholz und A.T. Graham, Wiley-VCH, 1998 beschrieben.

Weiterhin kann es vorteilhaft sein, wenn zwischen dem Weiterbehandlungsbereich und dem Trocknungsbereich noch ein Nachvernetzungsreaktor angeordnet ist, so dass die wasserabsorbierenden Polymergebilde noch vor dem Eintritt in den Weiterbehandlungsbereich oberflächennachvernetzt werden können. Geeignete Nachvernetzungsreaktoren sind beispielsweise im Absatz 3.2.8.1 in "Modern Superabsorbent Polymer Technology" von F.L. Buchholz und A.T. Graham, Wiley-VCH, 1998 beschrieben.

Der Weiterbehandlungsbereich umfasst vorzugsweise einen sich drehenden Behälter, wobei als sich drehenden Behälter diejenigen Behälter bevorzugt sind, die bereits eingangs im Zusammenhang mit dem erfindungsgemäßen Verfahren beschrieben worden sind.

Gemäß einer besonders bevorzugten Ausführungsform der erfindungsgemäßen Vorrichtung umfasst der Weiterbehandlungsbereich mindestens einen dem drehbaren Behälter vorgelagerten, weiteren Mischer, wobei es sich um diesen weiteren Mischer vorzugsweise um einen Kelley-Mischer, DRAIS-Turbulenzmischer, Lödigemischer, Ruberg-Mischer, Schneckenmischer, Tellermischer, Wirbelschichtmischer oder einen kontinuierlich arbeitenden senkrechten Mischer, in denen das Polymergebilde mittels rotierender Messer in schneller Frequenz gemischt wird (Schugi-Mischer), handelt. In diesem vorgelagerten Mischer können die wasserabsorbierenden Polymergebilde bereits vor Eintritt in den sich drehenden Behälter mit einem Modifizierungsmittel in Kontakt gebracht werden.

Einen Beitrag zur Lösung der eingangs genannten Aufgaben leistet auch ein Verfahren zur Herstellung eines Superabsorbers, bei dem eine vorstehend beschriebene Vorrichtung eingesetzt wird.

Einen weiteren Beitrag zur Lösung der eingangs genannten Aufgabe leistet ein Superabsorber, der durch das eingangs beschriebene, erfindungsgemäße Verfahren erhältlich ist.

Einen weiteren zur Lösung der eingangs beschriebenen Aufgaben liefert ein Verbund, beinhaltend die erfindungsgemäßen Superabsorber bzw. die durch das erfindungsgemäße Verfahren erhältlichen Superabsorber und ein Substrat. Es ist dabei bevorzugt, dass die erfindungsgemäßen Superabsorber und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher den erfindungsgemäßen Superabsorber in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 $cm^3$, vorzugsweise mindestens 0,1 $cm^3$ und am meisten bevorzugt mindestens 0,5 $cm^3$ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist. Der Offenbarungsgehalt der WO 02/056812 A1, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung mindestens einer der Eingangs genannten Aufgaben liefert ein Verfahren zur Herstellung eines Verbundes, wobei die erfindungsgemäßen Superabsorber bzw. die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung mindestens einer der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung mindestens einer der Eingangs genannten Aufgaben liefern chemische Produkte beinhaltend die erfindungsgemäßen Superabsorber oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Superabsorber oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung mindestens einer der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nun anhand nicht limitierender Figuren und Beispiele näher erläutert:
Es zeigt die Figur 1 eine besondere Ausgestaltung der erfindungsgemäßen Vorrichtung zur Herstellung von Superabsorbern im Querschnitt.

Es zeigt die Figur 2 eine weitere Ausgestaltung der erfindungsgemäßen Vorrichtung zur Herstellung von Superabsorbern im Querschnitt.

Es zeigt die Figur 3 eine besondere Ausgestaltung eines Wehrs als Drehscheibenwehr im Querschnitt.

Es zeigt die Figur 4 eine besondere Ausgestaltung des erfindungsgemäßen Verfahrens zur Herstellung von Superabsorbern.

Es zeigt die Figur 5 eine besondere Ausgestaltung eines Wehrs als Vertikalwehr im Querschnitt.

Es zeigt die Figur 6 eine besondere Ausgestaltung eines Wehrs als Mehrfachvertikalwehr im Querschnitt.

Figur 1 zeigt eine besondere Ausgestaltung der erfindungsgemäßen Vorrichtung zur Herstellung von Superabsorbern im Querschnitt. Im Polymerisationsbereich 1 wird aus einer wässrigen Monomerlösung beinhaltend teilneutralisierte Acrylsäure, mindestens einen Vernetzter sowie Initiatoren durch radikalische Polymerisation ein Polymergel gebildet, welches anschließend, gegebenenfalls nach einer vorhergehenden Zerkleinerung des Gels, in einem Trocknungsbereich 2 vorzugsweise bis zu einem Wassergehalt von weniger als 10 Gew.-% getrocknet wird. Das auf diese Weise erhaltene, wasserabsorbierende Vorprodukt kann dann, gegebenenfalls nach weiterem Zermahlen und Absieben auf eine bestimmte Partikelgrößenfraktion, in einem Nachvernetzungsreaktor 3 zunächst nachvernetzt werden, denkbar ist aber auch, das wasserabsorbierende Produkt direkt dem Weiterbehandlungsbereich zugeführt.

Der Weiterbehandlungsbereich 4 kann einen weiteren, vorzugsweise heizbaren Mischer 5 umfassen, in dem das wasserabsorbierende Vorprodukt mit einem Modifizierungsmittel, vorzugsweise einem Modifizierungsmittel, welches, wenn es nicht als Reinsubstanz zugesetzt wird, vorzugsweise in Form einer wässrigen Lösung, einer wässrigen Dispersion oder einer wässrigen Emulsion vorliegt, vermischt werden. Denkbar ist aber auch, das Modifizierungsmittel erst in dem sich drehenden Behälter 6 über die Zuführung 13 zuzusetzen. Gegebenenfalls kann das Modifizierungsmittel anteilig in einem vorgeschalteten Mischer 5 und anteilig über die Zuführung 13 zugesetzt werden.

In dem sich drehenden Behälter 6 wird das mit dem Modifizierungsmittel in Kontakt gebrachte, wasserabsorbierende Polymergebilde durch Mischen weiterbehandelt. Der sich drehende Behälter umfasst einen vorderen Kopfbereich 7, in dem sich auch der Produkteinlass 10 befindet, einen Mantelbereich 8 und einen hinteren Kopfbereich 9, in dem sich auch der Produktauslass 11 befindet. Im Inneren des sich drehenden Behälters sind Mischhilfen in Form von Schaufeln 12 fest an der Innenseite des sich drehenden Behälters angeordnet, die ein Durchmischen des wasserabsorbierenden Polymergebildes bei horizontaler Drehung des Behälters 6 (siehe der Pfeil rechts in Figur 1) ermöglichen. Die Drehung des Behälters wird durch die Krafteinwirkung der Motoren 14 ermöglicht.

Gemäß einer besonders bevorzugten Ausführungsform der erfindungsgemäßen Vorrichtung umfasst diese im Auslassbereich 10 ein Wehr 15 als Teil einer Auslasseinheit, dessen Querschnittsfläche reguliert werden kann (siehe hierzu auch die Figur 4).

Figur 2 zeigt eine weitere Ausgestaltung der erfindungsgemäßen Vorrichtung zur Herstellung von Superabsorbern im Querschnitt. Gemäß dieser besonderen Ausgestaltung umfasst der Weiterbehandlungsbereich zusätzlich eine Lanze 16, die mittels des Stellmotors 17 entlang der Rotationsachse des sich drehenden Behälters 6 in den Behälter 6 herein- und herausgeführt werden kann. An dieser Lanze 16 kann beispielsweise ein Austragselement 18 angebracht sein, welches den Austrag des Produktes über den Auslass 11 fördern kann. Außerdem ist der Produkteinlass 10 als geneigtes Lenkmittel wie ein Rohr oder eine Rutsche ausgestaltet. Der Grad der Neigung wie die gesamte Ausgestaltung des Produkteinlasses 10 sollte so gewählt werden, dass ein Anhaften des in den drehbaren Behälter 6 Eingebrachten bzw. dessen Verklumpen möglichst vermieden wird. Hierzu trägt auch eine möglichst Totraum-arme Gestaltung des Einlasses 10 bei. Außerdem können in dem Einlass 10 ein Förderelement 101 wie ein Rüttler, Förderband oder Schieber oder eine Kombination aus mindestens zwei davon vorgesehen sein.

Figur 3 zeigt eine besondere Ausgestaltung eines Wehrs 15 im Querschnitt. gemäß dieser besonderen Ausführungsform des Wehr besteht dieses aus einem unteren und einem oberen Kreissegment (151, 152), wobei das obere Kreissegment 152 um den Mittelpunkt des aus dem oberen Kreissegment 152 und dem unteren Kreissegment 151 gebildeten Kreises gedreht werden kann. Diese Drehung kann beispielsweise über eine Lanze 153, die mittels eines Stellmotors 154 bewegt werden kann, erfolgen. Wird das obere Kreissegment 152 gedreht, so entsteht ein Spalt 155, über den das wasserabsorbierende Produkt aus dem sich drehenden Behälter ausgetragen werden kann. Über die Breite des Spaltes kann somit die Menge an austretendem Material reguliert werden.

Figur 4 zeigt eine besondere Ausgestaltung des erfindungsgemäßen Verfahrens zur Herstellung von Superabsorbern. Gemäß dieser Ausführungsform wird ein wasserabsorbierendes Polymergebilde in einen sich drehenden Behälter 4 eingebracht, wobei das wasserabsorbierende Polymergebilde im Kopfbereich des sich drehenden Behälters 4 über eine erste Zuführung 12 mit einem ersten Modifizierungsmittel in Kontakt gebracht wird. Handelt es sich bei diesem ersten Modifizierungsmittel um einen Oberflächennachvernetzer, so kann es vorteilhaft sein, wenn der sich drehende Behälter 4 stromabwärts von der Zuführung des Oberflächennachvernetzers einen heizbaren Bereich 19 umfasst, in dem das wasserabsorbierende Polymergebilde erhitzt wird, um die Nachvernetzung zu bewirken. Das nachvernetzte Produkt passiert dann eine weitere Zuführung 20, in der ein weiteres Modifizierungsmittel zugesetzt werden kann, wobei dann beim weiteren Durchtritt des wasserabsorbierenden Polymergebildes eine entsprechende Weiterbehandlung durch die Drehung des Behälters und das daraus resultierende Vermischen des mit dem weiteren Modifizierungsmitteln in Kontakt gebrachten Superabsorbers erfolgt.

Figur 5 zeigt ein Vertikalwehr als weitere Ausgestaltung eines erfindungsgemäßen Wehrs 15. Ein in dem Ausgangsöffnungsbereich des drehbaren Behälters 6 vorgesehenes Auslassöffnungslager 21 nimmt eine darin drehbar gelagerte tellerförmige Auslassplatte 22 mit einer rechteckigen Austrittsöffnung 25 auf. Dieses weist einen beweglich gelagerten und an einer Fläche der Auslassplatte 22 schlüssig anliegenden recheckigen Auslassschieber 23 auf. Ein Auslassschieberantrieb 24 ist zum Bewegen des Auslassschiebers 23 vorgesehen. In Teil a. verschließt der Auslassschieber 23 die Austrittsöffnung 25, in Teil b. ist der Auslassschieber 23 verschoben und gibt die Auslassöffnung 25 frei, sodass sich der drehbare Behälter 26 bis zur unteren Marke der Auslassöffnung 25 entleeren kann.

Figur 6 zeigt ein Mehrfachvertikalwehr mit zwei Vertikalwehren als weitere Ausgestaltung eines erfindungsgemäßen Wehrs 15. Unter Bezugnahme auf die Ausführungen zu Figur 5 wird ergänzend ausgeführt, dass die in Figur 5 für das Einzelwehr geltende Beschreibung auch auf die zwei Vertikalwehre zutrifft, wobei das zweite Wehr durch die mit Apostroph versehenen Bezugszeichen gekennzeichneten Funktionen beschrieben wird. Wie auch für das Vertikalwehr nach Figur 5 ist es auch bei dem Mehrfachvertikalwehr bevorzugt, dass, bezogen auf eine durch den Maximaldurchmesser der Auslassplatte 22 sich ergebenden Wehrhöhenlinie 26, eine sich aus der Oberkante der freigebbaren Austrittsöffnung 25 ergebende Wehrhöhe 27 mindestens 30 %, vorzugsweise mindestens 50 % und besonders bevorzugt mindestens 60 % der Höhe der Wehrhöhenlinie 26 ausmacht.

TESTMETHODEN

Allgemein

Sofern nicht nachfolgend andere Testmethoden vorgegeben werden, wird auf die dem Fachmann allgemein bekannten und üblich erscheinenden Testmethoden zurückgegriffen, wobei insbesondere Testmethoden der EDANA (European Diaper and Nonwoven Association) Anwendung finden, die allgemein als "ERT-Methoden" angegeben werden.

Bestimmung der "Free Swell Rate" (FSR)

Diese die Absorptionsgeschwindigkeit angebende Testmethode wurde nach der in US 5,149,335 (Kellenberger et al.) beschriebenen Methode bestimmt.

Bestimmung des Staubanteils

Der Staubanteil wird mit einem Gerät der Firma AnaTec Deutschland GmbH, Deutschland des Typs *"Dust MON L"* bestimmt. Hierzu wird eine Probe von 30,00 g in ein Trichterrohr gegeben. Zu Beginn der Messung öffnet sich eine Trichterklappe automatisch und die Probe fällt in ein Staubreservoir. Nun wird die Verringerung eines Laserstrahls (Abnahme der Transmission) durch die Staubbildung gemessen. Dieser Wert dient der Bestimmung des Staubanteils. Der Staubanteil (STZ) ergibt sich aus einem Maximalwert am Beginn der Messung, und einem nach 30 Sekunden gemessenen Staubwert zur Bestimmung des schwebenden Anteils. Somit ergibt sich der Staubanteil aus der Summe aus Maximalwert und Staubwert.

Bestimmung des Wassergehaltes

Zur Bestimmung des Wassergehaltes wird das WDS 400 System 239 der Firma Sartorius verwendet. Das Messgerät WDS 400 wird durch ein Computerprogramm (WDS 400 Version 2.1.8) betrieben. Als Standard wird die Chemikalie Natriumwolframat-2-Hydrat verwendet. Zunächst wird Gasdurchfluss des Gerätes anhand einer Skala überprüft und auf 2 ccm/min eingestellt. Das WDS 400 System wird vor einer Messung ausgeheizt. Vor dem Ausheizen werden im Ofen befindliche Probenschiffchen mit einer Pinzette entfernt. Der weitere Ausheizvorgang wird von dem Computerprogramm gesteuert. Nach dem Ausheizen auf 400°C und einer Abkühlung auf unter 40°C wird eine Tara- und Standardmessung durchgeführt. Dazu wird zunächst ein Probenschiffchen mit einer Pinzette in das Gerät eingesetzt und dann wird die Tara eines Schiffchens für den Standard bestimmt. Dieser Messvorgang wird ebenso von dem Computerprogramm gesteuert, wobei es zu einer Temperaturerhöhung kommt. Nach diesem Messvorgang wird das WDS System wieder auf unter 40°C abgekühlt und das Schiffchen entfernt. In das Computerprogramm wird die genaue Einwaage (ca. 20 mg) des anschließend zu untersuchenden Produktes eingegeben. Nachdem das Schiffchen erneut mit der Pinzette eingesetzt wurde, wird die Messung gestartet. Das Computerprogramm steuert den vorgegebenen Zeitverlauf der Temperatur. In einem ersten Zeitabschnitt in dem Bereich von t = 0 bis t = 2 Min. beträgt die Temperatur 80 °C. In einen zweiten Zeitabschnitt in dem Bereich zwischen t = 2 Min. und t = 4 Min. wird die Temperatur gleichmäßig von 80°C auf 200°C erhöht. In einem dritten Zeitabschnitt in dem Bereich von t = 4 Min. bis t = 7 Min. wird die Temperatur konstant auf 200°C gehalten. In einem vierten Zeitabschnitt in dem Bereich zwischen t = 7 Min. bis t = 8 Min. wird die Temperatur gleichmäßig von 200°C auf 300 °C erhöht. Danach wird die Temperatur in einem fünften Zeitabschnitt bis zum Ende der Messung, das bei t = 25 min liegt, konstant auf 300 °C gehalten. Die Messergebnisse werden von dem Computerprogramm ausgewertet und durch einen Strom-Zeit-Diagramm mittels einer Kurve dargestellt. Die Kurve zeigt die zu einem bestimmten Zeitpunkt verdampfte Menge an Wasser, die zu dem dann gemessen Strom proportional ist. Das Integral unter der Kurve von t = 0 bis $t = t_1$ ist proportional zu dem Oberflächenwasser, wobei $t_1$ das relative Minimum vor dem letzten Maximum der gesamten Kurve ist. Das Kernwasser ergibt sich aus dem Integral der Kurve von $t_1$ bis zum Ende der Messung nach 25 Minuten, gerechnet von der ersten Temperaturerhöhung $T_1$.

FFC Wertmessung

Der FFC-Wert gibt Auskunft über die Fließeigenschaften eines Schüttgutes in einem Silo. Für die Messung wird das Ringschergerät RST-01.01 der Firma Dr. Ing. Dietmar Schulze Schüttgutmesstechnik in D-38302 Wolfenbüttel verwendet. Das RST-Gerät ist zur Steuerung und zur Ausgabe von Messergebnissen mit einem mit der Software RST Control 95 programmierten Computer und einem Drucker verbunden. Das Ringschergerät besitzt für Standard-Produktanalysen folgende Einstellungen: Beim Anscheren wird ein Druck von 500 Pa verwendet. Beim Abscheren werden Drücke von 100 Pa, 250 Pa, 400 Pa und 100 Pa verwendet. Nachdem die Geräte - Standardscherzelle mit Deckel auf das Gerät gesetzt ist, werden die Nullwerte der Kraftmessung geprüft. In dem Computerprogramm wird die gewünschte produktspezifische Messmethode ausgewählt werden. Dies geschieht zunächst mit einer Standardmessung (Bedingungen wie Oben beschrieben). Dann wird die Scherzelle mit wasserabsorbierenden Polymergebilde als Schüttgut befüllt, wobei auf eine glatte Oberfläche geachtet wird. Danach wird die Scherzelle zusammen mit dem Schüttgut gewogen. In das Computerprogramm wird das Gewicht der Scherzelle inklusive Schüttgut eingegeben. Bei der Standardmessung wird beim Anscheren ein Druck von 500 Pa verwendet. Beim Abscheren wird ein Druck von 100 Pa, 250 Pa, 400 Pa und 100 Pa verwendet. Nachdem die Messung durchgeführt ist, werden die Messergebnisse durch das Computerprogramm ermittelt, und gemäß Seiten 5 und 6 der Anleitung von Herrn Dr. Ing. Dietmar Schulze unter "HOME - www.dietmarschulze.de", Stand 12. März 2004 ausgewertet.

Wandreibungswinkel

Die Wandreibung beschreibt die Reibung zwischen einem Schüttgut und einem verwendeten Wandmaterial. Die Messung wird zwischen 18 - 23°C Raumtemperatur durchgeführt. Zur Messung der Wandreibung wird ein Ringschergerät der Firma Dr. Ing. Dietmar Schulze Schüttgutmesstechnik in D-38302 Wolfenbüttel eingesetzt. Der Bodenring des Schergerätes wird durch das Wandmaterial das untersucht werden soll ersetzt. Dann wird die Messzelle mit wasserabsorbierendes Polymergebilde als Schüttgut in das Schergerät befüllt. Eine senkrecht zu dem zu untersuchenden Wandmaterial wirkende durch das Ringschergerät erzeugte Kraft verursacht die Normalspannung $\sigma_w$. Dann wird die bei wirkender Normalspannung zum Verschieben des Schüttgutes notwendige Scherkraft gemessen, die senkrecht zur Normalkraft wirkt und die die Schubspannung $\tau_w$ erzeugt. Diese Schubspannung wird bestimmt, indem die gemessene Schubkraft durch die Größe der Wandfläche geteilt wird, auf die die Schubkraft wirkt. Auf diese Weise wird für eine gegebene Normalspannung $\sigma_w$ die zugehörige Schubspannung $\tau_w$ angegeben. Aus diesem Wertepaar kann der Wandreibungswinkel $\varphi_x$ bestimmt werden, indem die Größen wie folgt in Beziehung gesetzt werden:

$$\tan(\varphi_x) = \tau_W / \sigma_W$$

und gemäß Seiten 11 und 12 der Anleitung von Herrn Dr. Ing. Dietmar Schulze unter "HOME - www.dietmar-schulze.de", Stand 12. März 2004 ausgewertet.

BEISPIELE

Beispiel 1

Ein Wasserabsorbierendes Polymergebilde (das bei der Firma Stockhausen GmbH Krefeld, erhältliche Produkt Favor® SXM 9300) wurde in einem Drais-Mischer vom Typ K-TT 4E mit einer durchschnittliche Verweilzeit im Mischer von ca. 30-60 sec, der bei 900 Umdrehungen pro Minuten betrieben wurde, mit 2 Gew.-% Wasser als Modifizierungsmittel versetzt. Das erhaltene Polymergebilde mit dem Modifizierungsmittel ist als erdfeucht zu beschreiben. Anschließend wird das so behandelte Polymergebilde mit einem Produkteintrag von 80 kg/Std., in den Lindor-Mischer überführt, der mit 4,75 Umdrehungen pro Minute betrieben wurde. Während der Befüllphase war das Wehr geschlossen. Anschließend wurde das Wehr schrittweise geöffnet, bis sich ein Produktaustrag von 1,82 kg/min eingestellt hat. Das so erhaltene Modifizierte Polymergebilde ist frei fließfähig und unterscheidet sich äußerlich nicht von Ausgangsprodukt. Die Eigenschaften vor und nach der Behandlung sind in Tabelle 1 als Mittel aus je drei Probenentnahmen dargestellt.

Tabelle 1:

| Produkt | Oberflächewasser | Kernwasser | Gesamtwasser | Staubanteil | FSR |
|---|---|---|---|---|---|
| SXM 9300 | 2,3% (39%)* | 3,5% (61%) | 5,8% | 1,2 | 0,2 |
| Ex Trommelmischer | 3,5% (47%) | 3,8% (53%) | 7,3% | 0,7 | 0,2 |
| *= entspricht Anteil am Gesamtwasser | | | | | |

Durch die Behandlung nach dem erfindungsgemäßen Verfahren lässt sich eine sehr gleichmäßige Verteilung des Modifizierungsmittels bei gringerer Staubneigung und gleich bleibenden Quelleigenschaften erreichen.

Beispiel 2

Wie in Beispiel 1 angegeben, wurde ein Wasserabsorbierendes Polymergebilde (das bei der Firma Stockhausen GmbH Krefeld, erhältliche Produkt Favor® SXM 9155) mit 2 Gew. % Wasser als Modifizierungsmittel im Drais-Mischer behandelt und dem Lindor-Mischer zugeführt. Das Wehr wurde nach der Befüllphase so eingestellt, dass sich ein Gleichgewicht zwischen zufließenden und abfließenden Produkt im Lindor-Mischer von ca. 80 kg/Std. einstellte. In Tabelle 2 ist der kleinteilige Bereich der Teilchengrößenverteilung gemäß Siebanalyse vor und nach der Behandlung angegeben.

Tabelle 2

| Teilchengröße / Produkt | Favor® SXM 9155 | Ex Trommelmischer |
|---|---|---|
| >300μm | 13,5% | 13,5% |
| >200μm | 14,7% | 13,4% |
| >100μm | 7,2% | 7,0% |
| <100μm | 0,7% | 0,9% |

Es ist aus Tabelle 2 erkennbar, dass die Menge an Teilchen mit kleinen Teilchengrößen im Wesentlichen konstant bleibt. Hierdurch wird das besonders schonende Mischen des erfindungsgemäßen Verfahrens gezeigt.

Beispiel 3

Ein Wasserabsorbierendes Polymergebilde (das bei der Firma Stockhausen GmbH Krefeld, erhältliche Produkt Favor® SXM 9155) wurde mit 2 Gew. % Wasser als Modifizierungsmittel, mittels im Lindor-Mischer vorhandene Düse, versetzt. Das Wehr wurde nach der Befüllphase so eingestellt das sich ein Gleichgewicht zwischen zufließenden und

abfließenden Produkt im Lindor-Mischer von ca 80kg/Std. einstellte. In Tabelle 3 ist der kleinteilige Bereich der Teilchengrößenverteilung gemäß Siebanalyse vor und nach der Behandlung angegeben.

Tabelle 3

| Teilchengröße / Produkt | Favor® SXM 9155 | Ex Trommelmischer |
|---|---|---|
| >300μm | 12,9% | 12,7% |
| >200μm | 12,0% | 11,3% |
| >100μm | 4,6% | 4,2% |
| <100μm | 0,2% | 0,4% |

Es ist aus Tabelle 3 erkennbar, dass die Menge an Teilchen mit kleinen Teilchengrößen im Wesentlichen konstant bleibt. Hierdurch wird das besonders schonende Mischen des erfindungsgemäßen Verfahrens gezeigt.

Beispiel 4

Wie in Beispiel 1 angegeben, wurde ein Wasserabsorbierendes Polymergebilde (das bei der Firma Stockhausen GmbH Krefeld, erhältliche Produkt Favor® SXM 9155) mit 10 Gew. % einer 50% igen Zitronensäure-Lösung, so wie 1,0% einer Fällungskieselsäure (Sipernat), als Modifizierungsmittel im Drais-Mischer behandelt und dem Lindor-Mischer zugeführt. Das Wehr wurde nach der Befüllphase so eingestellt das sich ein Gleichgewicht zwischen zufließenden und abfließenden Produkt im Lindor-Mischer von ca. 80kg/Std. einstellte. Der so erhaltene Modifizierte Superabsorber ist fließfähig. Wegen der hohen Flüssigkeitsmenge und der klebrigen Eigenschaft von der Zitronensäure, ist ohne eine sehr gute Durchmischung im Lindor-Mischer das leicht angequollene Polymergebilde, welches extrem klumpig, feucht und klebrig ist, in einem herkömmlichen Mischer nicht weiter in einer großtechnisch sinnvollen Weise zu verarbeiten.

Beispiel 5

Wie in Beispiel 1 angegeben, wurde ein Wasserabsorbierendes Polymergebilde (das bei der Firma Stockhausen GmbH Krefeld, erhältliche Produkt SXM 9155) mit 5 Gew. % einer 33%igen Tegosorb® A30-Lösung, sowie 0,5% einer Fällungskieselsäure (Sipernat), als Modifizierungsmittel im Drais-Mischer behandelt und dem Lindor-Mischer zugeführt. Das Wehr wurde nach der Befüllphase so eingestellt das sich ein Gleichgewicht zwischen zufließenden und abfließenden Produkt im Lindor-Mischer von ca. 80kg/Std. einstellte. Folgende Kendaten wurden vor und nach dem Lindor-Mischer erhalten. Wegen der hohen Flüssigkeitsmenge mit leicht öliger Konsistenz ist ohne eine sehr gute Durchmischung im Lindor-Mischer das leicht angequollene Polymergebilde, welches extrem klumpig und feucht ist, in einem herkömmlichen Mischer nicht weiter in einer großtechnisch sinnvollen Weise zu Verarbeiten. Die Eigenschaften des so erhaltenen Superabsorbers sind in Tabelle 4 angegeben.

Tabelle 4

| FFC-Wert: | Vor Lindor-Mischer | Nach Lindor-Mischer |
|---|---|---|
| Probe 1 | 4,5 | 6,2 |
| Wandreibungswinkel | 11,4 | 11,8 |

Es ist erkennbar, dass eine deutliche Verbesserung der durch den FFC-Wert gekennzeichneten Fließfähigkeit bei weiterhin guter Wandreibung erzielt wurde.

**BEZUGSZEICHENLISTE**

| | |
|---|---|
| 1 | Polymerisationsbereich |
| 2 | Trocknungsbereich |
| 3 | Oberflächennachvernetzungsreaktor |
| 4 | Weiterbehandlungsbereich |
| 5 | weiterer Mischer |

6 sich drehender Behälter
7 vorderer Kopfbereich des sich drehenden Behälters
8 Mantelbereich des sich drehenden Behälters
9 hinterer Kopfbereich des sich drehenden Behälters
10 Produkteinlass
101 Förderelement
11 Produktauslass
12 Schaufel
13 Zuführung für ein Modifizierungsmittel
14 Motor
15 Wehr
151 unteres Kreissegment des Wehrs
152 oberes Kreissegment des Wehrs
153 Lanze zur Regelung des Wehrs
154 Motor zur horizontalen Bewegung der Lanze 143
155 geöffneter Spalt im Wehr, durch den Superabsorber aus dem sich drehenden Behälter austreten kann
16 Lanze zum Verschieben eines Austragselements im Inneren des sich drehenden Behälters
17 Motor zur horizontalen Bewegung der Lanze 15
18 Austragselement
19 heizbarer oder kühlbarer Bereich
20 weitere Zuführung für ein Modifizierungsmittel
21 Auslassöffnungslager
22 Auslassplatte
23 Auslassschieber
24 Auslassschieberantrieb
25 Austrittsöffnung
26 Wehrhöhenlinie
27 Wehrhöhe

**Patentansprüche**

1. Ein Verfahren zur Herstellung eines Superabsorbers, beinhaltend als Schritte:

a) Bereitstellen eines oberflächennachvernetzten, wasserabsorbierenden Polymergebildes;
b) in Kontakt bringen des wasserabsorbierenden Polymergebildes mit einem Modifizierungsmittel, , ;
c) Weiterbehandeln des mit dem Modifizierungsmittel in Kontakt gebrachten, wasserabsorbierenden Polymergebildes;

wobei zumindest die Weiterbehandlung im Verfahrensschritt c) kontinuierlich, zumindest teilweise in einem sich drehenden Behälter erfolgt, der in eine Drehung um eine horizontal durch die Mitte des Behälters verlaufende Achse versetzbar ist, in welchem sich abgesehen von einer möglicherweise beweglichen Ein- und Auslasseinrichtung keine beweglichen und gegenläufige Teile befinden, der Behälter bis maximal 80 % seiner größten Querschnittsfläche befüllt ist und der sich drehende Behälter von dem wasserabsorbierenden Polymergebilde durchströmt wird, wobei die durchschnittliche Verweilzeit der wasserabsorbierenden Polymergebilde in dem sich drehenden Behälter in einem Bereich von 5 bis 300 Minuten liegt;
und wobei das eingesetzte Modifizierungsmittel ausgewählt ist aus der Gruppe bestehend aus:

ii) einer wässrigen Lösung umfassend Wasser und mindestens ein in Wasser gelöstes, wasserlösliches Polymer, besonders bevorzugt ein Polyethylenglykol, insbesondere mit einem Molekulargewicht in einem Bereich von 300 bis 15.000 g/mol;
iii) einer wässrigen Suspension umfassend Wasser, eine protonierte, organische Säure, vorzugsweise Zitronensäure, sowie ein anorganisches Feinteilchen, vorzugsweise eine SiO-Verbindung, besonders bevorzugt eine Fällungskieselsäure;
iv) einer wässrigen Lösung umfassend Wasser sowie mindestens ein organisches Metallsalz, vorzugsweise Aluminiumlactat;
vi) einer wässrigen Lösung umfassend Wasser sowie ein anorganisches Metallsalz, vorzugsweise Aluminiumsulfat;

viii) einem anorganischen Pulver, vorzugsweise ein anorganisches Metallsalz, besonders bevorzugt Aluminiumsulfat, Aluminiumchlorid oder Aluminiumphosphat, oder eine SiO-Verbindung, vorzugsweise eine pyrogene Kieselsäure oder eine Fällungskieselsäure;
ix) einem organischen Pulver, vorzugsweise auf Polysacchariden basierende Pulver, beispielsweise Carboxyzellulosepartikel, oder Cyclodextrin-Partikel;
x) einer wässrigen Dispersion umfassend Wasser, mindestens ein anorganisches oder organisches Metallsalz, vorzugsweise Aluminiumsulfat oder Aluminiumlactat, sowie mindestens ein Oxid eines Metalls, vorzugsweise ein Zinkoxid;
xii) einer wässrigen Lösung umfassend Wasser und mindestens ein Polykation, vorzugsweise ein Polydiallyldimethylammoniumchlorid;
xiv) einer wässrigen Dispersion umfassend Wasser und ein in Wasser dispergiertes, thermoplastisches Polymer;
xvi) einer wässrigen Lösung bzw. einer wässrigen Dispersion umfassend eine Verbindung der Struktur II, Wasser sowie gegebenenfalls ein anorganisches Feinteilchen, vorzugsweise eine SiO- Verbindung, besonders bevorzugt eine Fällungskieselsäure, wobei Struktur II wie folgt definiert ist:

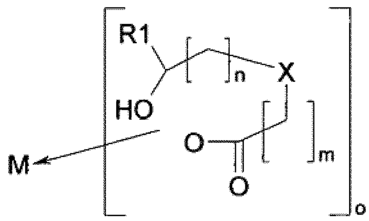

Struktur II

mit

R1 als einen $C_1$-$C_{20}$-, vorzugsweise einen $C_1$-$C_{15}$- sowie darüber hinaus bevorzugt einen $C_3$-$C_{10}$-Kohlenwasserstoff;
X als nicht vorhanden (in diesem Fall besteht eine direkte Kohlenstoff-Kohlenstoffbindung zwischen dem Kohlenstoff in $[]_n$ und dem Kohlenstoff in $[]_m$) oder ein in einem Bereich von 1 bis 5, vorzugsweise in einem Bereich von 1 bis 3 Doppelbindungen oder besonders bevorzugt eine einzige Doppelbindung aufweisendes, im Falle von mindestens 2 Doppelbindungen, vorzugsweise konjugiertes Doppelbindungssystem;
M als ein geladenes oder ungeladenes, vorzugsweise geladenes Metall; m in einem Bereich von 1 bis 15, vorzugsweise in einem Bereich von 3 bis 12 und darüber hinaus bevorzugt in einem Bereich von 5 bis 9;
n in einem Bereich von 1 bis 5, vorzugsweise in einem Bereich von 1 bis 3 und darüber hinaus bevorzugt 1;
o in einem Bereich von 1 bis 4, vorzugsweise in einem Bereich von 1 bis 3 und besonders bevorzugt in einem Bereich von 1 bis 2 sowie darüber hinaus bevorzugt 2;

xvii) Wasser, insbesondere destilliertes Wasser, mittel Ionenaustauscher entionisiertes Wasser oder aber Leitungswasser;
xviii) wässrigen Lösungen oder wässrigen Suspensionen basierend auf organischen Feststoffen, vorzugsweise auf Polysacchariden basierende Pulver, beispielsweise Carboxyzellulosepartikel, oder Cyclodextrin-Partikel;
xix) einer wässrigen Lösung eines wasserlöslichen Silbersalzes;
xx) einer wässrigen Dispersion einer wasserunlöslichen Silbersalzes..

**2.** Verfahren nach Anspruch 1, wobei das im Verfahrensschritt a) bereitgestellte, wasserabsorbierende Polymergebilde einen nach der hier angegebenen Methode bestimmten Wassergehalt von höchstens 20 Gew.-%, bezogen auf das Gesamtgewicht des wasserabsorbierenden Polymergebildes, aufweist.

**3.** Verfahren nach einem der vorhergehenden Ansprüche, wobei der Behälter mindestens teilweise trommelartig ausgebildet ist.

## Claims

**1.** A process for producing a superabsorbent, comprising, as steps:

a) providing a surface postcrosslinked, water-absorbing polymer structure;
b) contacting the water-absorbing polymer structure with a modifying agent;
c) subjecting the water-absorbing polymer structure that has been contacted with the modifying agent to further treatment;

wherein at least the further treatment in process step c) is effected continuously, at least partly in a rotating vessel that can be set in a rotation about an axis that runs horizontally through the middle of the vessel, in which, apart from a possibly movable inlet and outlet device, there are no moving and counter-rotating parts, the vessel has been filled up to 80% of its greatest cross-sectional area and the water-absorbing polymer structure flows through the rotating vessel, where the average residence time of the water-absorbing polymer structure in the rotating vessel is within a range of 5 to 300 minutes; and wherein the modifying agent used is selected from the group consisting of:

ii) an aqueous solution comprising water and at least one water-soluble polymer that has been dissolved in water, more preferably polyethylene glycol, especially having a molecular weight within a range from 300 to 15 000 g/mol;
iii) an aqueous suspension comprising water, a protonated organic acid, preferably citric acid, and inorganic fines, preferably an SiO compound, more preferably a precipitated silica;
iv) an aqueous solution comprising water and at least one organic metal salt, preferably aluminium lactate;
vi) an aqueous solution comprising water and an organic metal salt, preferably aluminium sulfate;
viii) an inorganic powder, preferably an inorganic metal salt, more preferably aluminium sulfate, aluminium chloride or aluminium phosphate, or an SiO compound, preferably a fumed silica or a precipitated silica;
ix) an organic powder, preferably powder based on polysaccharides, for example carboxycellulose particles, or cyclodextrin particles;
x) an aqueous dispersion comprising water, at least one inorganic or organic metal salt, preferably aluminium sulfate or aluminium lactate, and at least one oxide of a metal, preferably a zinc oxide;
xii) an aqueous solution comprising water and at least one polycation, preferably a polydiallyl-dimethylammonium chloride;
xiv) an aqueous dispersion comprising water and a thermoplastic polymer dispersed in water;
xvi) an aqueous solution or an aqueous dispersion comprising a compound of the structure II, water and optionally inorganic fines, preferably an SiO compound, more preferably a precipitated silica, where structure II is defined as follows:

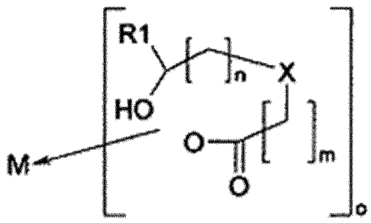

Structure II

with

R1 as a $C_1$-$C_{20}$ hydrocarbyl radical, preferably a $C_1$-$C_{15}$ and further preferably a $C_3$-$C_{10}$ hydrocarbyl radical;
X as absent (in this case there is a direct carbon-carbon bond between the carbon in $[\ ]_n$ and the carbon in $[]_m$) or a double bond system having within a range from 1 to 5, preferably within a range from 1 to 3, double bonds or more preferably a single double bond, preferably a conjugated double bond system in the case of at least 2 double bonds;
M as a charged or uncharged, preferably charged, metal; m within a range from 1 to 15, preferably within a range from 3 to 12 and further preferably within a range from 5 to 9;
n within a range from 1 to 5, preferably within a range from 1 to 3 and further preferably 1;
o within a range from 1 to 4, preferably within a range from 1 to 3 and more preferably within a range from 1 to 2, and further preferably 2;

xv
ii) water, especially distilled water, ion exchanger-deionized water or else tap water;
xviii) aqueous solutions or aqueous suspensions based on organic solids, preferably powders based on polysaccharides, for example carboxycellulose particles, or cyclodextrin particles;
xix) an aqueous solution of a water-soluble silver salt;
xx) an aqueous dispersion of a water-insoluble silver salt.

**2.** Process according to Claim 1, wherein the water-absorbing polymer structure provided in process step a) has a water content, determined by the method specified herein, of not more than 20% by weight, based on the total weight of the water-absorbing polymer structure.

**3.** Process according to either of the preceding claims, wherein the vessel is at least partly in the form of a drum.

## Revendications

**1.** Procédé pour la préparation d'un superabsorbant, comportant comme étapes :

a) la préparation d'une structure polymère absorbant l'eau postréticulée en surface ;
b) la mise en contact de la structure polymère absorbant l'eau avec un agent de modification ;
c) le traitement ultérieur de la structure polymère absorbant l'eau mise en contact avec l'agent de modification ;

le traitement ultérieur dans l'étape de procédé c) ayant lieu en continu, au moins partiellement dans un récipient rotatif, qui peut être mis en rotation autour d'un axe s'étendant horizontalement à travers le centre du récipient, dans lequel ne se trouvent pas de pièces mobiles et en sens contraire, à l'exception d'un dispositif d'entrée et de sortie éventuellement mobile, le récipient étant rempli jusqu'à au maximum 80% de sa surface transversale la plus grande et le récipient en rotation étant traversé par la structure polymère absorbant l'eau, la durée de séjour moyenne de la structure polymère absorbant l'eau dans le récipient en rotation se situant dans une plage de 5 à 300 minutes ; et l'agent de modification utilisé étant choisi dans le groupe constitué par :

ii) une solution aqueuse comprenant de l'eau et au moins un polymère soluble dans l'eau, dissous dans l'eau, de manière particulièrement préférée un polyéthylèneglycol, présentant en particulier un poids moléculaire dans la plage de 300 à 15.000 g/mole ;
iii) une suspension aqueuse comprenant de l'eau, un acide organique protoné, de préférence l'acide citrique, ainsi que de fines particules inorganiques, de préférence un composé à base de SiO, de manière particulièrement préférée une silice précipitée ;
iv) une solution aqueuse comprenant de l'eau ainsi qu'au moins un sel métallique organique, de préférence le lactate d'aluminium ;
vi) une solution aqueuse comprenant de l'eau ainsi qu'un sel métallique inorganique, de préférence le sulfate d'aluminium ;
viii) une poudre inorganique, de préférence un sel métallique inorganique, de manière particulièrement préférée le sulfate d'aluminium, le chlorure d'aluminium ou le phosphate d'aluminium, ou un composé à base de SiO, de préférence une silice pyrogène ou une silice précipitée ;
ix) une poudre organique, de préférence des poudres à base de polysaccharides, par exemple des particules de carboxycellulose ou des particules de cyclodextrine ;
x) une dispersion aqueuse comprenant de l'eau, au moins un sel métallique inorganique ou organique, de préférence le sulfate d'aluminium ou le lactate d'aluminium, ainsi qu'au moins un oxyde d'un métal, de préférence un oxyde d'étain ;
xii) une solution aqueuse comprenant de l'eau et au moins un polycation, de préférence un chlorure de polydiallyldiméthylammonium ;
xiv) une dispersion aqueuse comprenant de l'eau et un polymère thermoplastique dispersé dans l'eau ;
xvi) une solution aqueuse ou une dispersion aqueuse comprenant un composé de structure II, de l'eau ainsi que le cas échéant de fines particules inorganiques, de préférence un composé à base de SiO, de manière particulièrement préférée une silice précipitée, la structure II étant définie comme suit :

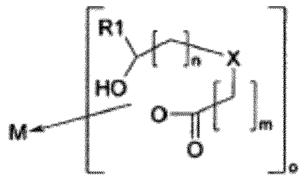

structure II

où

R1 représente un hydrocarbure en $C_1$-$C_{20}$, de préférence en $C_1$-$C_{15}$ et plus préférablement en $C_3$-$C_{10}$ ;
X n'est pas présent (dans ce cas, il existe une liaison carbone-carbone directe entre le carbone dans $[]_n$ et le carbone dans $[]_m$) ou représente un système à doubles liaisons, présentant des doubles liaisons dans une plage de 1 à 5 doubles liaisons, de préférence dans une plage de 1 à 3 doubles liaisons ou de manière particulièrement préférée une seule double liaison, de préférence conjugué dans le cas d'au moins 2 doubles liaisons ;
M représente un métal chargé ou non chargé, de préférence chargé ;
m se situe dans une plage de 1 à 15, de préférence dans une plage de 3 à 12 et plus préférablement dans une plage de 5 à 9 ;
n se situe dans une plage de 1 à 5, de préférence dans une plage de 1 à 3 et vaut plus préférablement 1 ;
o se situe dans une plage de 1 à 4, de préférence dans une plage de 1 à 3 et de manière particulièrement préférée dans une plage de 1 à 2 et vaut de préférence 2 ;

xvii) de l'eau, en particulier de l'eau distillée, de l'eau désionisée par un échangeur ionique ou encore de l'eau courante ;
xviii) des solutions aqueuses ou des suspensions aqueuses à base de solides organiques, de préférence des poudres à base de polysaccharides, par exemple des particules de carboxycellulose ou des particules de cyclodextrine ;
xix) une solution aqueuse d'un sel d'argent soluble dans l'eau ;
xx) une dispersion aqueuse d'un sel d'argent insoluble dans l'eau.

**2.** Procédé selon la revendication 1, la structure polymère absorbant l'eau préparée dans l'étape de procédé a) présentant une teneur en eau, déterminée selon un procédé indiqué dans la description, d'au plus 20% en poids, par rapport au poids total de la structure polymère absorbant l'eau.

**3.** Procédé selon l'une quelconque des revendications précédentes, le récipient étant conçu au moins partiellement en forme de tambour.

Fig. 1

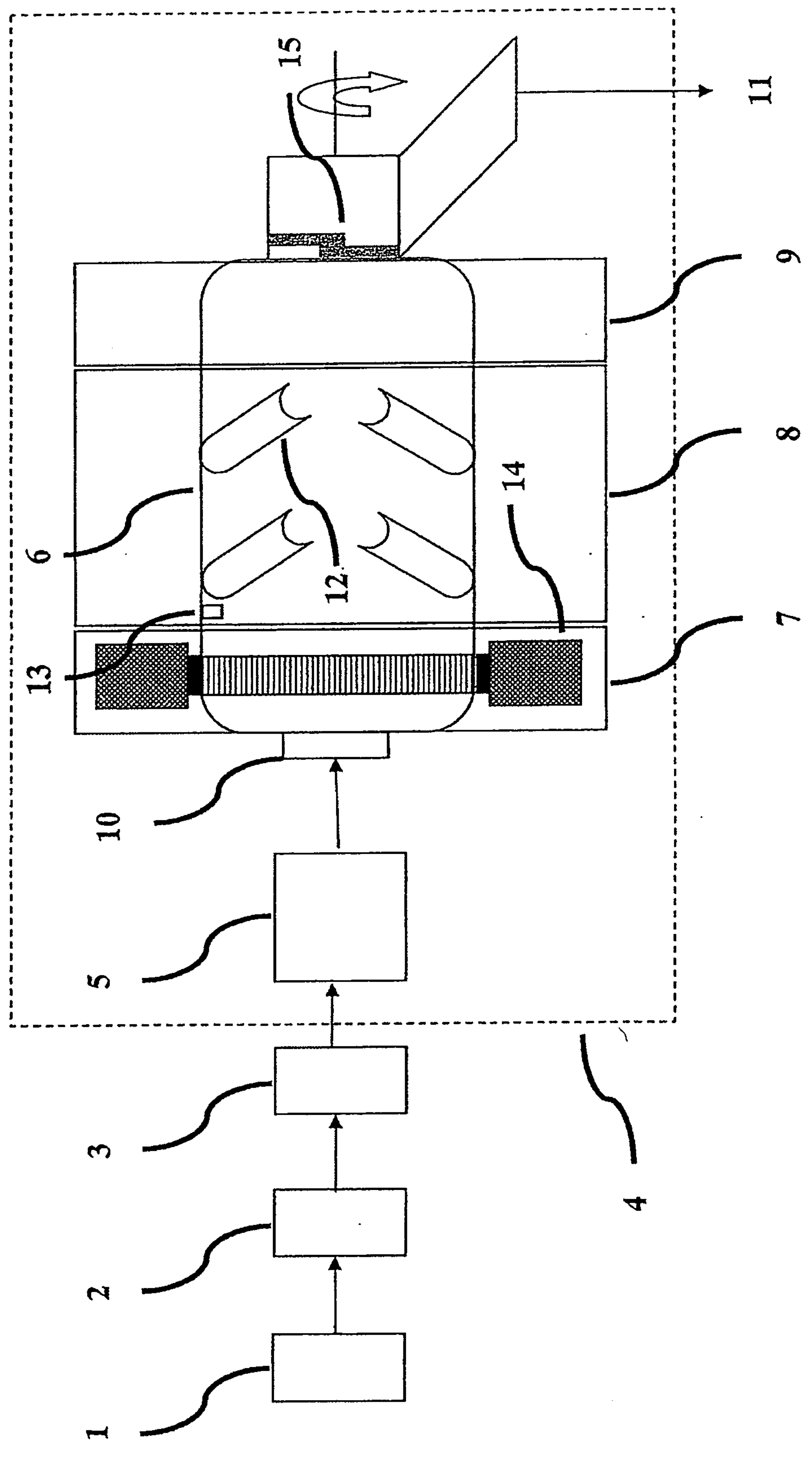

Fig. 2

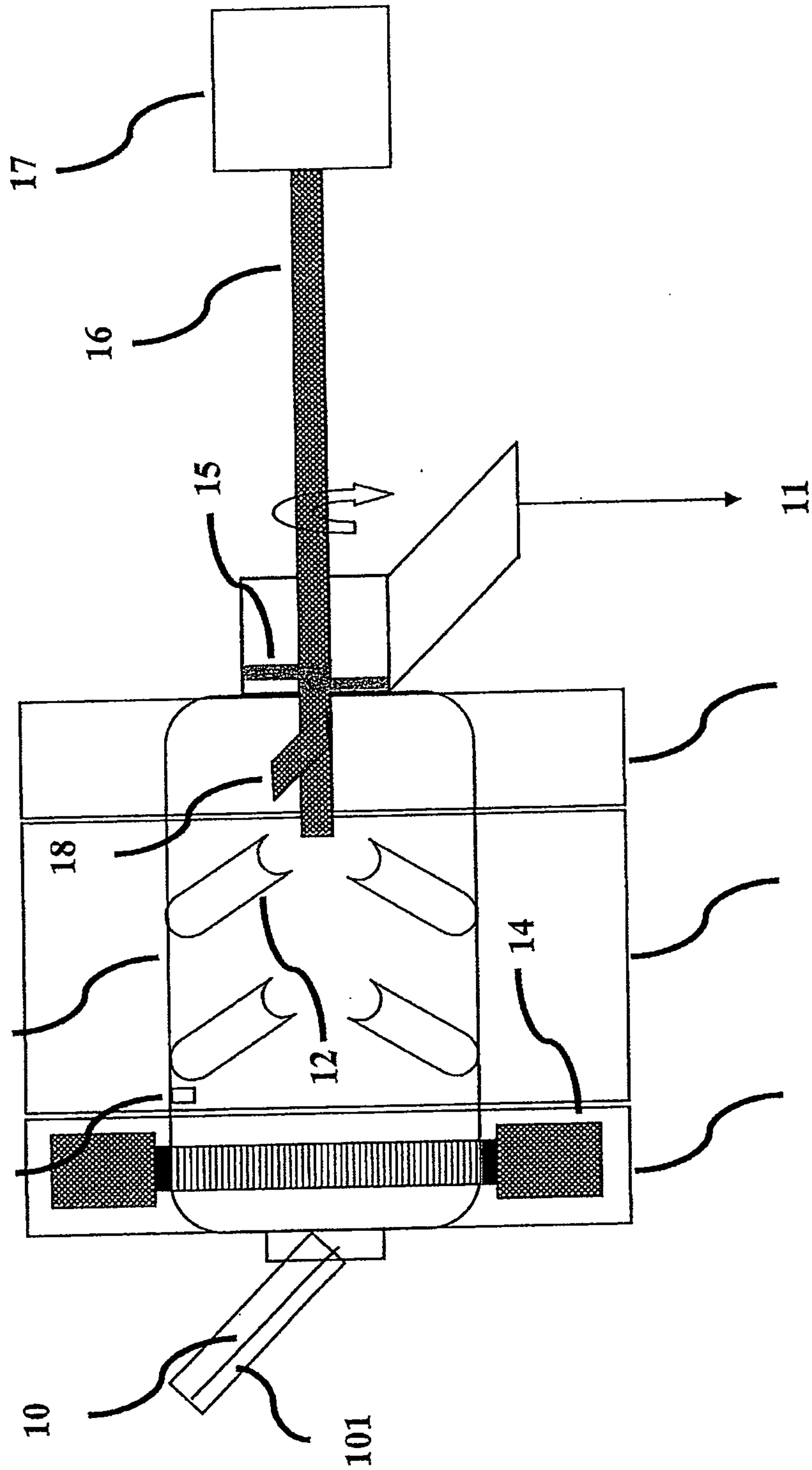

Fig. 3

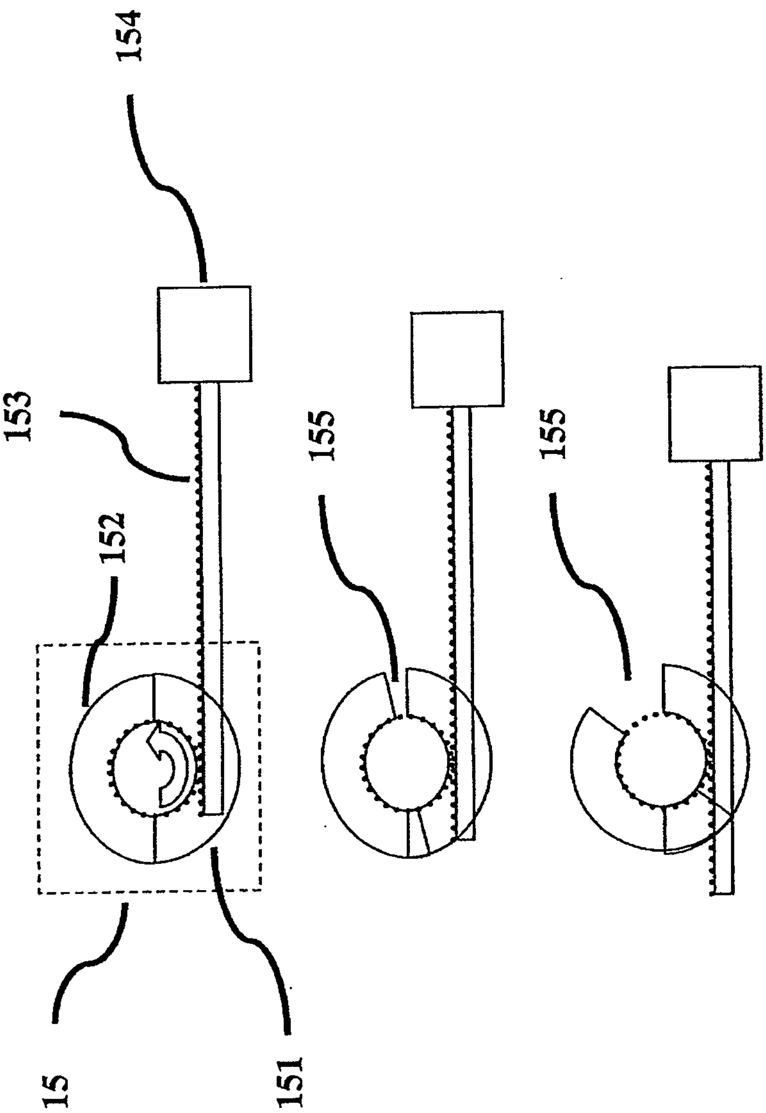

Fig. 4

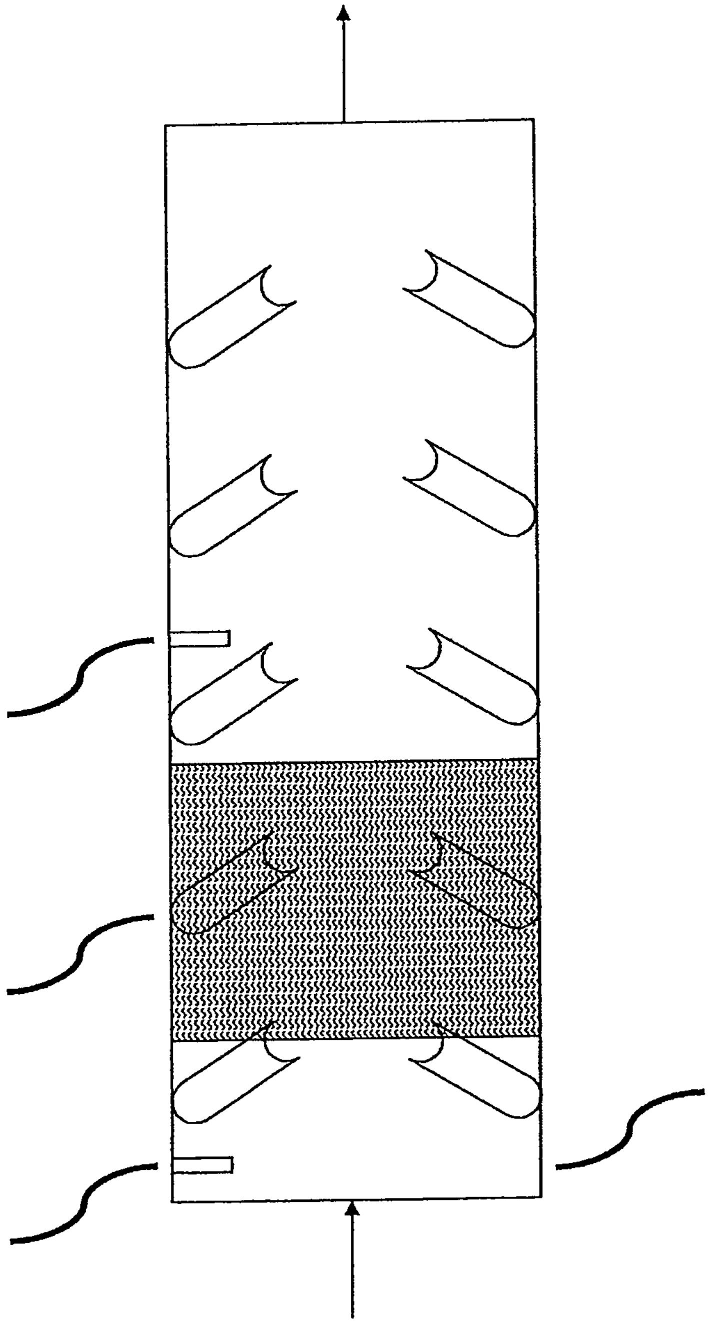

Fig. 5

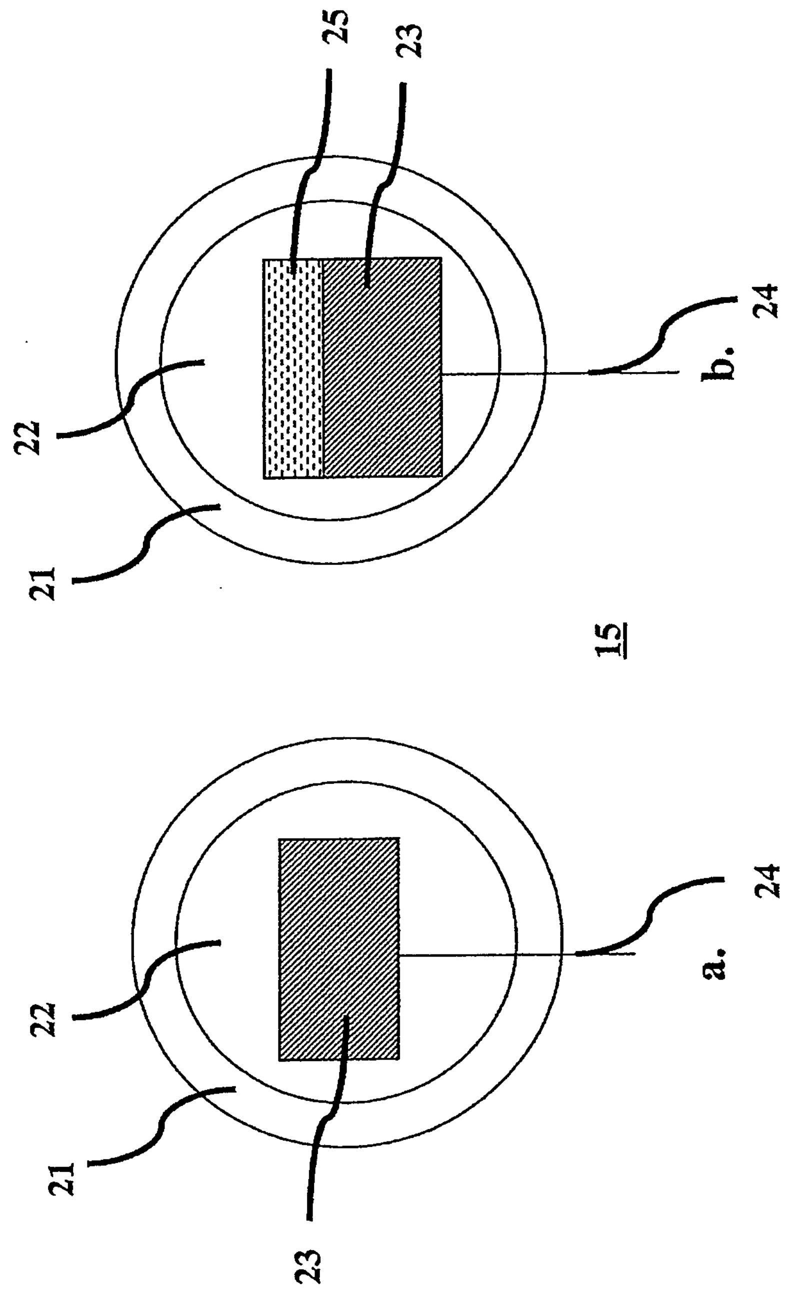

Fig. 6

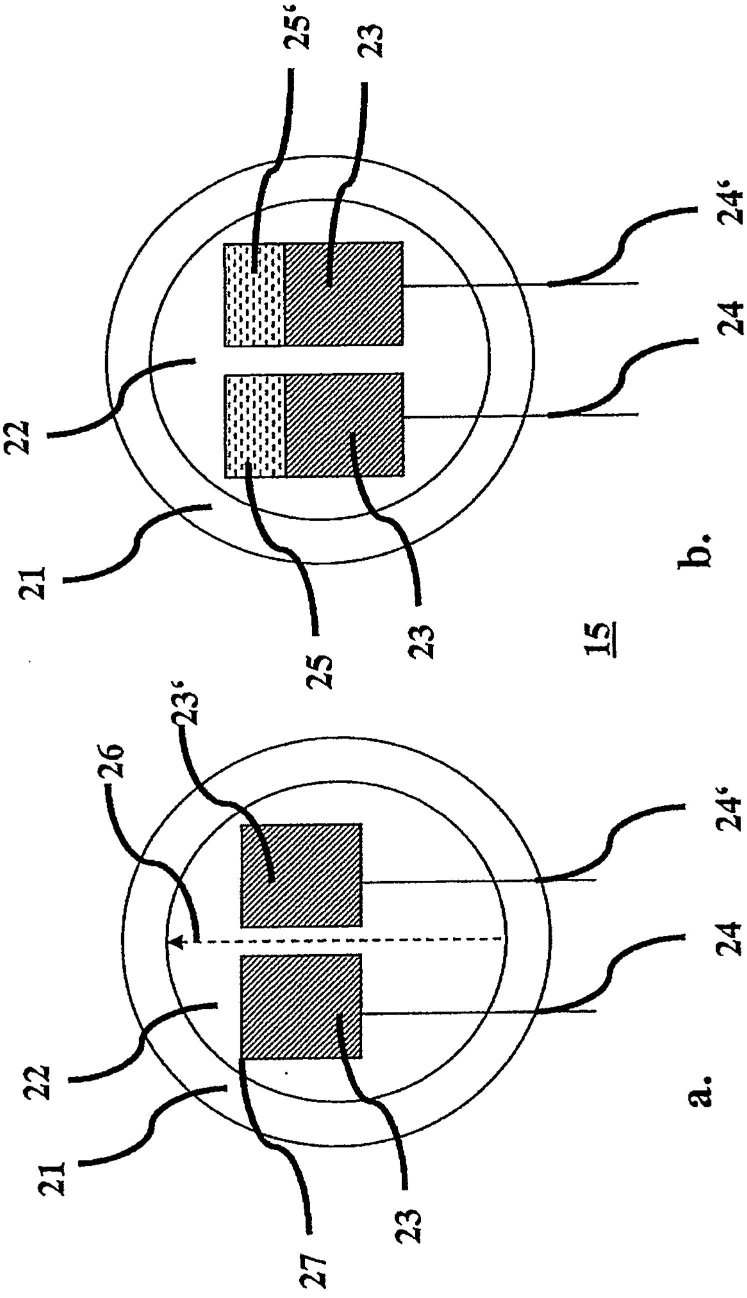

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS2612846 A
- DE 19529348 A1
- WO 9934843 A1
- WO 2004037903 A2
- US 4286082 A
- DE 2706135
- US 4076663 A
- DE 3503458
- DE 4020780
- DE 4244548
- DE 4323001
- DE 4333056
- DE 4418818
- US 4340706 A
- DE 3713601
- DE 2840010
- WO 9605234 A1
- DE 10334286 A
- WO 2005044900 A
- WO 2005011860 A1
- US 20060029567 A1
- WO 03090799 A
- WO 02056812 A1
- US 5149335 A, Kellenberger


### In der Beschreibung aufgeführte Nicht-Patentliteratur


- **F.L. BUCHHOLZ ; A.T. GRAHAM.** Modern Superabsorbent Polymer Technology. Wiley-VCH, 1998
- **JACEK K. DUTKIEWICZ.** *New Approach to Odor and pH Control in Personal Care,* 29. Oktober 2006
- **HERRN DR. ING.** HOME - www.dietmar-schulze.de. *Dietmar Schulze,* Marz 2004, 5, , 6
- **HERRN DR.** HOME - www.dietmar-schulze.de. *Ing. Dietmar Schulze,* 12. Marz 2004